# EUROPEAN PATENT APPLICATION

(11) **EP 2 338 514 A1**
(43) Date of publication of application: **29.06.2011**
(21) Application number: 10192703.6
(22) Date of filing: 20.03.2008
(51) Int. Cl.: A61K 39/395, A61K 38/20

(54) **Combined human IL-18 and pazopanib cancer treatment**

(30) Priority: 23.03.2007 US 896855 P; 26.07.2007 US 952002 P
(62) Divisional of application: 08744104.4
(71) Applicant: Glaxosmithkline LLC, Philadelphia, PA 19102 (US)
(72) Inventor: Haskova, Zdenka, King of Prussia, PA 19406 (US); Jonak, Zdenka, Ludmila, King of Prussia, PA 19406 (US); Trulli, Stephen, H., King of Prussia, PA 19406 (US); Whitacre, Margaret, N., King of Prussia, PA 19406 (US)
(74) Representative: Reddish, Anna

(57) **Abstract**

The present invention relates generally to the use of human IL-18 combinations in the treatment of various forms of solid tumors and lymphomas. In particular, the present invention relates to combinations of human IL-18 with chemotherapeutc agents, specifically pazopanib.

## Description

### CROSS-REFERENCE TO RELATED APPLICATIONS

This application claims priority to two earlier US provisional applications, US Application No., 60/952,002, filed on 26 July 2007, and US Application No. 60/896,855, filed on 23 March 2007.

### FIELD OF INVENTION

The present invention relates generally to the use of IL-18, also known as interferon-γ-inducing factor (IGIF), in combination with a monoclonal antibody that is expressed on the surface of a cancer cell, or in combination with a chemotherapeutic agent, to treat cancer.

### BACKGROUND OF THE INVENTION

Interleukin-18 (IL-18) is a potent cytokine that plays a role in both innate and acquired immune responses. In pre-clinical studies, IL-18 induces synthesis of IFN-γ by T cells and natural killer (NK) cells, augments the cytolytic activity of NK cells and cytotoxic T lymphocytes (CTL), promotes differentiation of activated CD4 T cells into helper effector cells and induces immunological memory. Based upon a broad spectrum of immuno-stimulatory properties, IL-18 has been studied in a variety of pre-clinical tumor models. The anti-tumor activity of IL-18, used as a monotherapy, was observed in tumors that were immunogenic. The most potent anti-tumor effects were observed in an advanced tumor (>100 cm³) model of MOPC-315 plasmacytoma (highly immunogenic tumor). As tumors are usually non-immunogenic, the focus of pre-clinical studies was on combination therapies of IL-18 with monoclonal antibodies or chemotherapeutic agents. These studies showed the benefit of combining two different agents, each with different mechanism of tumor killing, resulting in synergistic anti-tumor activity.

Active human IL-18 contains 157 amino acid residues. It has potent biological activities, including induction of interferon-γ-production by T cells and splenocytes, enhancement of the killing activity of NK cells and promotion of the differentiation of naive CD4⁺T cells into Th1 cells. In addition, human IL-18 augments the production of GM-CSF and decreases the production of IL-10. CD4⁺ T cells are the central regulatory elements of all immune responses. They are divided into two subsets, Th1 and Th2. Each subset is defined by its ability to secrete different cytokines. Interestingly, the most potent inducers for the differentiation are cytokines themselves. The development of Th2 cells from naive precursors is induced by IL-4. Prior to the discovery of IL-18, IL-12 was thought of as the principal Th1 inducing cytokine.

Th1 cells secrete IL-2, interferon-γ, and TNF-β. Interferon-γ, the signature Th1 cytokine, acts directly on macrophages to enhance their microbiocidal and phagocytic activities. As a result, the activated macrophages can efficiently destroy intracellular pathogens and tumor cells. The Th2 cells produce IL-4, IL-5, IL-6, IL-10 and IL-13, which act by helping B cells develop into antibody-producing cells. Taken together, Th1 cells are primarily responsible for cell-mediated immunity, while Th2 cells are responsible for humoral immunity.

Based upon a broad spectrum of immunostimulatory properties, IL- 18 has been studied in a variety of preclinical tumor models. The anti-tumor activity of IL-18, used as a monotherapy, was observed in tumors that were immunogenic. The most potent anti-tumor effects were observed in the advanced tumor (>100 cm³) model of MOPC-315 plasmacytoma (highly immunogenic tumor). In this model, daily administration of murine IL-18 (5mg/Kg) for approximately 30 days resulted in a reproducible tumor regressions and cure. Rechallenge with parental tumor resulted in tumor rejection, suggesting induction of immunological memory. Additional evidence for involvement of cellular immunity in this model comes from experiments conducted in severe combined immunodeficient mice (SCIDs) bearing advanced MOPC-315 tumors that failed to regress when using a similar schedule of IL-18. Further support for IL-18 mediated cellular immunity also comes from immunohistochemistry performed on established MOPC-315 tumors in control and IL-18 treated mice. This demonstrated increased cellular infiltrates consisting of CD8⁺ T lymphocytes, NK cells, activated macrophages, and dendritic cells in the IL-18 treated animals relative to controls. *In vitro,* PBMCs or spleen cells from animals treated with IL-18 showed NK and CTL cytotoxicity against the tumor. In addition, it seems that an intact Fas/Fas ligand pathway is beneficial to anti-tumor response.

Rituximab is a chimeric monoclonal antibody that consists of a murine antigen binding site that recognizes the human CD20 antigen fused to the human IgG1 constant region. Rituximab, as a single agent, has significant activity in indolent NHL. In the pivotal single-arm clinical study of 166 patients with relapsed or refractory indolent NHL, the overall response rate was 48% and the complete response (CR) rate was 6%. McLaughlin, et al., J. Clin. Oncol. 16:2825-2833 (1998). In previously untreated patients with indolent NHL, Rituximab therapy has an overall response rate of 64 to 73% and CR rate of 15 to 26%. Hainsworth, et al., Blood 95:3052-3056 (2000); Colombat, et al., Blood 97:101-106 (2001). Moreover, multiple randomized Phase III studies have shown that addition of Rituximab to conventional chemotherapy improves the survival of patients with NHL. Marcus, et al., Blood 105:1417-1423 (2005); Marcus, et al., Blood 104:3064-3071 (2004); Hiddemann, et al., Blood 106:3725-3732 (2005); Feugier, et al., J. Clin. Oncol. 23:4117-4126 (2005). However, due to higher toxicity with chemotherapy, monotherapy with Rituximab is still considered an option in patients with indolent lymphoma.

Research is ongoing to determine ways of enhancing the anti-tumor activity and improve the efficacy of Rituximab. Several mechanisms may contribute to the efficacy of Rituximab in vivo. Binding of Rituximab to CD20 on the surface of lymphoma cells can trigger intracellular signaling pathways leading to apoptosis or programmed cell death. Shan, et al., Blood 91:1644-1652 (1998); Pedersen, et al., Blood 99:1314-1319 (2002). Moreover, Rituximab can activate complement species, causing complement-dependent cytolysis. Cragg, et al., Blood 101:1045-1052 (2003); Manches, et al., Blood 101:949-954 (2003). However, accumulating evidence suggests that ADCC plays a dominant role in elimination of tumor cells after administration of Rituximab. Manches, *et al., supra;* Golay, et al., Haematologica 88:1002-1012 (2003); Clynes, et al., Nat. Med. 6:443-446 (2000). ADCC is triggered when the constant (Fc) region of an antibody binds to Fc receptors on the surface of effector cells, such as NK cells or cells of monocyte/macrophage lineage.

In a murine model of human B cell lymphoma, the efficacy of Rituximab was abrogated in mice lacking activating Fc receptors. In contrast, monoclonal antibody therapy was enhanced in mice lacking inhibitory Fc receptors. Fc receptor-bearing effector cells were critical for the efficacy of Rituximab in this model. A major activating Fc receptor in humans is CD16 (FcγRIIIA), which is expressed by NK cells and monocytes. A polymorphism in the human FcγRIIIA gene at position 158 (phenylalanine versus valine) has been shown to correlate with response to Rituximab. The 158VV homozygous genotype is associated with stronger IgG binding to and triggering of ADCC by human NK cells *in vitro* (Koene, et al., Blood 90:1109-1114 (1997); Dall'Ozzo, et al., Cancer Res. 64:4664-4669 (2004)), and is also associated with a higher rate of response after Rituximab therapy. Weng, et al., J. Clin. Oncol. 21:3940-3947 (2003); Cartron, et al., Blood 104:2635-2642 (2004). These data support the hypothesis that NK cell-mediated ADCC is important for the effectiveness of Rituximab therapy in patients with lymphoma.

One strategy for improving the efficacy of Rituximab is to administer cytokines that can cause the expansion and/or activation of Fc receptor-bearing effector cells, including NK cells and cells of monocyte/macrophage lineage. Phase I clinical trials have shown that Rituximab can be safely given in combination with IL-2, IL-12, or GM-CSF to patients with lymphoma. Rossi, et al., Blood 106:2760 (abst 2432) (2005); McLaughlin, et al., Ann. Oncol. 16 (Suppl 5):v68 (abstr 104) (2005); Ansell, et al., Blood 99:67-74 (2002); Eisenbeis, et al., Clin. Cancer Res. 10:6101-6110 (2004); Gluck, et al., Clin. Cancer Res. 10:2253-2264 (2004); Friedberg, et al., Br. J. Haematol. 117:828-834 (2002). Overall objective response rates of 22 to 79% and complete response rates of 5-45% were observed in these studies. In addition, biomarkers such as absolute NK counts and *ex vivo* ADCC activity correlated with response rates. Most of these studies included predominantly patients with relapsed and refractory disease and with aggressive lymphoma subtypes (DLBCL and mantle cell lymphoma). Relatively high objective response rates in these unfavorable patient populations indicate that combinations of cytokines and Rituximab are worthy of further investigation in B cell lymphoma.

### SUMMARY OF THE INVENTION

In one aspect, the present invention relates to a method of treating cancer in a patient in need thereof, comprising the step of: separately administering, either simultaneously, or sequentially, to the patient a composition comprising: (i) a human IL-18 polypeptide (SEQ ID NO:1) in combination with a carrier and; and (ii) a monoclonal antibody against an antigen that is expressed on the surface of a cancer cell, wherein the antibody has antibody-dependent-cell-mediated cytoxicity (ADCC) effector function, and further wherein the antibody is not an anti-CD20 antibody.

This first method may involve administering a composition comprising a monoclonal antibody against an antigen chosen from the group of: CD22, CD19, HER2, HER3, EGFR (Erbitux), and IGF-1R, AXL-1, FGFR, integrin receptors, CEA, CD44, VEGFR. In another aspect, the antigen is HER-2, and the monoclonal antibody is HERCEPTIN^{®}. Additionally, this method involves treating a cancer that is chosen from the group of: Hodgkin's lymphoma, B-cell non-Hodgkin's lymphoma, Burkitt lymphoma, T-cell Non-Hodgkin's lymphoma, AML, CLL, MM, other leukemias, ovarian cancer, breast cancer, lung cancer, sarcoma, bladder cancer, pancreatic cancer, thyroid cancer, hepatoma, gastric cancer, Wilms', neuroblastoma, glioblastoma and other brain tumors, colon cancer, rectal cancer, prostate cancer, melanoma, renal cell carcinoma, and skin cancers.

In a second aspect, this invention pertains to a method of treating cancer in a patient in need thereof, comprising the step of: separately administering, either simultaneously or sequentially, to the patient a composition comprising: (i) human IL-18 polypeptide (SEQ ID NO:1) in combination with a carrier; and (ii) a chemotherapeutic agent. The chemotherapeutic agent in this method may be chosen from the group of: doxil, topotecan, DNA-altering drugs (e.g., carboplatin), antimetabolites (e.g., gemcitabine), drugs that prevent cell division (e.g., vincristine) and anti-angiogenic agents (e.g., pazopanib). In this method, the cancer to be treated is chosen from the group of: Hodgkin's lymphoma, B-cell non-Hodgkin's lymphoma, T-cell Non-Hodgkin's lymphoma, breast cancer, lung cancer, sarcoma, bladder cancer, thyroid cancer, hepatoma, gastric cancer, Wilms' tumor, neuroblastoma, colon cancer, colorectal cancer, prostate cancer, melanoma, and renal cell carcinoma.

In another aspect, the invention provides a composition comprising a human IL-18 polypeptide (SEQ ID NO:1), and a monoclonal antibody against an antigen that is expressed on the surface of a cancer cell for use in the treatment of cancer, wherein the antibody has antibody-dependent cell-mediated cytotoxicity (ADCC) effector function, and wherein the antibody is not an anti-CD20 antibody. The composition comprising the hIL-18 polypeptide (SEQ ID NO: 1) and the antibody may be for administration separately to the patient, or optionally, simultaneously or sequentially.

In another aspect, the invention provides the use of a composition comprising a human IL-18 polypeptide (SEQ ID NO:1) and a monoclonal antibody against an antigen that is expressed on the surface of a cancer cell in the manufacture of a medicament for the treatment of cancer in a patient, wherein the monoclonal antibody has antibody-dependent cell-mediated cytotoxicity (ADCC) effector function, and wherein the antibody is not an anti-CD20 antibody. The hIL-18 polypeptide and the antibody may be for administration separately to the patient, optionally simultaneously or sequentially.

In another aspect, the invention provides the use of a composition comprising a human IL-18 polypeptide (SEQ ID NO:1) in the manufacture of a medicament for use in combination with a monoclonal antibody against an antigen that is expressed on the surface of a cancer cell for the treatment of cancer in a patient, wherein the monoclonal antibody has antibody-dependent cell-mediated cytotoxicity (ADCC) effector function, and wherein the antibody is not an anti-CD20 antibody.

In another aspect, the invention provides the use of a monoclonal antibody against an antigen that is expressed on the surface of a cancer cell in the manufacture of a medicament for use in composition comprising the combination with a human IL-18 polypeptide (SEQ ID NO:1) for the treatment of cancer in a patient, wherein the monoclonal antibody has antibody-dependent cell-mediated cytotoxicity (ADCC) effector function, and wherein the antibody is not an anti-CD20 antibody.

In another aspect, the invention provides a composition comprising: (i) a human IL-18 polypeptide (SEQ ID NO:1), and (ii) a chemotherapeutic agent for use in the treatment of cancer. The composition comprising the hIL-18 polypeptide (SEQ ID NO:1) and the chemotherapeutic agent may be for administration separately to the patient, optionally simultaneously or sequentially.

In another aspect, the invention provides the use of a composition comprising a human IL-18 polypeptide (SEQ ID NO:1) and a chemotherapeutic agent in the manufacture of a medicament for the treatment of cancer. The hIL-18 polypeptide (SEQ ID NO:1) and the chemotherapeutic agent may be for administration separately to the patient, optionally, simultaneously or sequentially.

In another aspect, the invention provides the use of a human IL-18 polypeptide (SEQ ID NO:1) in the manufacture of a medicament for use in combination with a chemotherapeutic agent for a composition to treat cancer.

In another aspect, the invention provides the use of a chemotherapeutic agent in the manufacture of a medicament for use in a composition comprising the combination with a human IL-18 polypeptide (SEQ ID NO:1) in the treatment of cancer.

In yet another aspect, the invention provides a method of treating cancer in a patient in need thereof, said method comprising the step of administering to the patient a composition comprising: human IL-18 (SEQ ID NO:1) in combination with a chemotherapeutic agent or a monoclonal antibody against an antigen that is expressed on the surface of a cancer cell, wherein the antibody has antibody-dependent-cell-mediated cytoxicity (ADCC) effector function, and further wherein the antibody is not an anti-CD20 antibody, whereby the treatment results in long-term survival and/or prevention of cancer reoccurrence and induction of immunological memory in the patient.

### BRIEF DESCRIPTION OF THE FIGURES

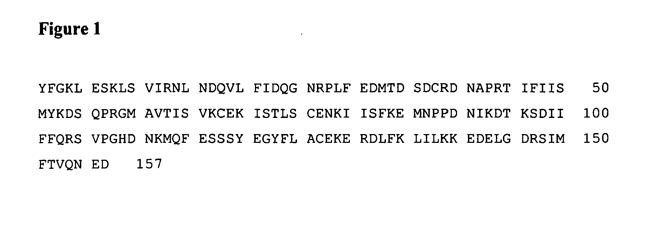

Figure 1 shows the amino acid sequence of native human IL-18 (SEQ ID NO:1).

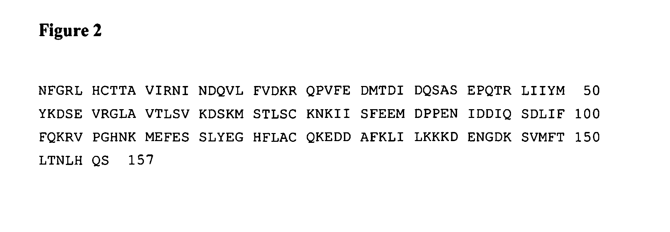

Figure 2 shows the amino acid sequence of murine IL-18 (SEQ ID NO:2).
Figure 3 shows the anti-tumor activity ofmIL-18 (SEQ ID NO:2) in combination with RITUXAN^{®} in a human B-cell lymphoma murine model.
Figure 4 shows the statistical significance when the data from Figure 3 are graphed and analyzed using GraphPad Prism^{®}. Specifically, this figure compares tumor volumes on day 19 post-implantation.
Figure 5 shows the tumor volume on day 25 post-implantation of the murine IL-18 (SEQ ID NO:2)/RITUXAN^{®} combination in a human B-cell lymphoma model.
Figures 6A and 6B shows median and mean tumor growth volume of the murine IL-18 (SEQ ID NO:2)/RITUXAN^{®} combination in a human B-cell lymphoma model.
Figures 7 and 8 show tumor volume on day 27 post-implantation of the murine IL-18 (SEQ ID NO:2)/RITUXAN^{®} combination in a human B-cell lymphoma model, versus either agent alone.
Figure 9 shows the EL-4 T-cell survival post-treatment of mIL-18 (SEQ ID NO:2) in combination with doxorubicin, versus both doxorubicin alone and IL-18 alone.
Figure 10 shows the survivor probability plot of the data demonstrated in Figure 9, which shows the relationship between dose of drug given and anti-tumor activity in the EL-4 T-cell lymphoma model.
Figures 11A and 11B show Facs analysis ofPBLs (Figure 11A) and splenocytes (Figure 11B) on day 13 after implantation of the doxorubicin/IL-I8 combination versus both mIL-18 (SEQ ID NO:2) alone and doxorubicin alone in the EL-4 T-cell lymphoma model.
Figure 12 demonstrates an NK cytotoxicity assay 21 hours post-treatment of doxorubicin/mIL-18 (SEQ ID NO:2) combination versus both IL-18 alone and doxorubicin alone in the EL-4 T-cell lymphoma model.
Figure 13 shows the effect of combination therapy with mIL-18 (SEQ ID NO:2) and HERCEPTIN^{®} on the growth of MOPC315 murine plasmocytoma in SCID mice in the MOPC315.D3j005 study. (Data expressed as mean +/-SD.)
Figure 14 shows the effect of combination therapy with mIL-18 (SEQ ID NO:2) and HERCEPTIN^{®} on the growth of MOPC315 murine plasmocytoma in SCID mice in the MOPC315.D3j005 study. (Data expressed as median +/-SD.)
Figure 15 shows statistical difference in tumor volume on day 24 post-implantation with the combination therapy ofmIL-18 (SEQ ID NO:2) and HERCEPTIN^{®} on the growth of MOPC315 murine plasmocytoma in SCID mice. (Data expressed as mean +/-SD.)
Figure 16 shows the tumor volume on day 24 post-implantation with the combination therapy ofmIL-18 (SEQ ID NO:2) and HERCEPTIN^{®} on the growth of MOPC315.D3j005 murine plasmocytoma in SCID mice. (Data expressed as median +/-SD.)
Figure 17 shows the effect of combination therapy with mIL-18 (SEQ ID NO:2) and HERCEPTIN^{®} on the growth of MOPC315 murine plasmocytoma in SCID mice in the MOPC315.D3j03 study. (Data expressed as mean +/-SD.)
Figure 18 shows the effect of combination therapy with mIL-18 (SEQ ID NO:2) and HERCEPTIN^{®} on the growth of MOPC315 murine plasmocytoma in SCID mice in the MOPC315.D3j03 study. (Data expressed as median +/-SD.)
Figure 19 shows the MOPC315 plasmocytoma volume on day 24 post-implantation in SCID mice from the combination therapy of mIL-18 (SEQ ID NO:2) and HERCEPTIN^{®} in the MOPC315.D3j03 study. (Data expressed as mean +/-SD.)
Figure 20 shows the MOPC315 plasmocytoma volume on day 24 post-implantation in SCID mice from the combination therapy of mIL-18 (SEQ ID NO:2) and HERCEPTIN^{®} in the MOPC315.D3j03 study. (Data expressed as median +/-SD.)
Figure 21 shows the effect of combination therapy of mIL-18 (SEQ ID NO:2) and 5-fluorouracil (5-FU) in a syngeneic murine Colo26 colon cancer model on day 24 after inoculation. (Data expressed as mean +/-SD.)
Figure 22 shows the effect of combination therapy of mIL-18 (SEQ ID NO:2) and 5-FU in a syngeneic murine Colo26 colon cancer model on day 24 after inoculation (data expressed as median +/-SD).
Figure 23 shows the effect of combination therapy of mIL-18 (SEQ ID NO:2) and 5-FU in a syngeneic murine Colo26 colon cancer model on day 24 after inoculation, and removing the control group for better view of statistical significance between IL-18 alone and the combination with 5-FU. (Data expressed as mean +/-SD.)
Figure 24 shows the effect of combination therapy of mIL-18 (SEQ ID NO:2) and 5-FU in a syngeneic murine Colo26 colon cancer model. (Data expressed as median +/-SD.)
Figure 25 shows the effect of combination therapy of mIL-18 (SEQ ID NO:2) and 5-FU in a syngeneic murine Colo26 colon cancer model. (Data expressed as mean +/-SD.)
Figure 26 shows the effect of combination therapy of mIL-18 (SEQ ID NO:2) and 5-FU in a syngeneic murine Colo26 colon cancer model on day 24 after inoculation. (Data expressed as Kaplan-Meyer survival curve.)
Figure 27 shows the effect of combination therapy of mIL-18 (SEQ ID NO:2) and pazopanib (GW786034), an inhibitor of VEGFR and PDGFR and c-kit tyrosine kinases, on tumor growth on day 32 post-implantation in an advanced syngeneic model of mouse renal carcinoma. (The c-Kit receptor belongs to type III tyrosine kinase receptor, which consists of an extracellular ligand binding domain and an intracellular kinase domain. The c-Kit receptor is expressed in a wide variety of normal and neoplastic tissues).
Figure 28 shows the effect of combination therapy of mIL-18 (SEQ ID NO:2) and pazopanib (GW786034) on tumor growth on day 32 post-implantation in an advanced syngeneic model of mouse renal carcinoma, but excludes the control group. This graph compares the statistical significance of the combination to monotherapy with IL-18 or pazopanib alone.
Figure 29 shows the body weight gain ofIL-2- treated immunodeficient mice that received adoptive transfer of cells from EL-4 tumor survivors or from naive controls.
Figure 30 shows the percent survival of Pfp/Rag2 recipient mice with IL-2 therapy after EL-4 tumor inoculation.

### DETAILED DESCRIPTION OF THE INVENTION

Since tumors are usually non-immunogenic the focus of pre-clinical studies is focused on combination therapies of IL-18 with chemotherapeutic agents or with monoclonal antibodies. Combining two different agents in a composition, each with different mechanism of tumor killing, results in synergistic anti-tumor activity. Four examples ofIL-18 combination therapies are presented below.

Example 1 focuses on the use of IL-18 in combination with RITUXAN^{®} in a human B-cell lymphoma. The aim of this study is to investigate whether the combination of IL-18 and RITUXAN^{®} in the human B cell lymphoma model offers a benefit over the monotherapy with IL-18, or RITUXAN^{®} alone. Rituximab is an approved chimeric monoclonal antibody that consists of a murine antigen binding site that recognizes the human CD20 antigen and a human IgG1 constant region. The mechanism of action that contributes to the efficacy of Rituximab *in vivo* includes induction of apoptosis upon binding to lymphoma cells that are CD20-positive, complement-dependent cytotoxicity (CDC) and antibody-dependent cell-mediated cytotoxicity (ADCC). Accumulating evidence suggests that ADCC plays a dominant role in the elimination of tumor cells after administration of Rituximab. ADCC is triggered when the constant (Fc) region of an antibody binds to Fc receptors on the surface of effector cells, such as natural killer (NK) cells, T-cells, or cells of monocyte/macrophage lineage. Since IL-18 augments and activates the ADCC effector cells, combination of these two reagents is expected to show synergy that would result in superior anti-tumor activity.

Rituximab (RITUXAN^{®}) is a chimeric monoclonal antibody that consists of a murine antigen binding site that recognizes the human CD20 antigen, fused to the human IgG1 constant region. CD20 antigen is expressed on malignant and non-malignant B lymphocytes. As a single agent, RITUXAN^{®} has significant activity in NHL. RITUXAN^{®} is commercially available.

Doxorubicin (adriamycin) is a chemotherapeutic agent that is commercially available, and is used for treatment of breast cancer, lymphomas, sarcoma, lung cancer, bladder cancer, thyroid, hepatoma, gastric cancer, Wilms' tumor, neuroblastoma, acute lymphocytic leukaemia (ALL), and ovarian cancers.

Example 2 shows the combination of IL-18 with doxorubicin in an EL-4 T-cell lymphoma model. The aim of this study was to investigate the combination of IL-18 with doxorubicin in the syngeneic EL-4 T-cell lymphoma tumor model, and to demonstrate the benefit of combination therapy over monotherapy with IL-18, or doxorubicin alone. This syngeneic model reveals the full benefits of IL-18 immunostimulatory activity on the host's immune cells. Since the two reagents have very different mechanisms of action, they can complement each other, resulting in increased anti-tumor activity. It is suggested that the chemotherapeutic agent provides the direct cytotoxicity, fragmentation and modulation of the tumor antigens, while IL-18 augments and activates the effector cells, resulting in superior antigen presentation and synergistic anti-tumor activity.

The combination of IL-18 with monoclonal antibodies in a composition, for example IL-18 with Rituximab (RITUXAN^{®}), showed synergistic anti-tumor activity in an advanced stage tumor model (SCID mouse xenograft). Rituximab is an approved chimeric monoclonal antibody that consists of a murine antigen binding site that recognizes the human CD20 antigen and human IgG1 constant region. Rituximab as a single agent has significant activity in indolent Non-Hodgkin's lymphoma. The mechanism of action that contributes to the efficacy of Rituximab *in vivo* includes induction of apoptosis upon binding to lymphoma cells that are CD20-positive, complement-dependent cytotoxicity (CDC) and antibody-dependent cell-mediated cytotoxicity (ADCC). The pre-clinical data shown in Example 1 demonstrates that combination of IL-18 and Rituximab results in synergistic anti-tumor activity. Since Rituximab is only binding to human tumor cells that express CD20, the assessment of anti-tumor activity was limited to SCID mouse xenograft models. It is believed that It is believed that the anti-tumor activity and synergy of IL-18 and Rituximab it due to NK cells that are activated in SCID mouse in response to IL-18 and to murine CDC. Since SCIDS have no capability to activate T-cells, the arm of potential CTL augmentation and memory generation can not be tested in this model.

The combination of IL-18 with chemotherapeutic agents in a composition will likely have beneficial therapeutic effect in treating various forms of cancer. For example, Example 2 shows that the combination of IL-18 with doxorubicin has synergistic anti-tumor activity in a syngeneic advanced EL4 T-cell lymphoma tumor model. This data suggests that IL-18's mechanism of action includes superior antigen presentation, expansion of anti-tumor CTLs and NK cells that play a key role in anti-tumor activity. Based upon these results, the combination of IL-18 with other chemotherapeutic agents will likely result in tumor regression, tumor cure and induction of immunological memory.

In clinical trials, IL-18 monotherapy was shown to be safe, well tolerated, and biologically active as measured by biomarker changes. Pre-clinically, IL-18 monotherapy worked only in immuno-sensitive tumor models. Non-immunogenic models showed anti-tumor activity only when IL-18 was combined with other anti-cancer agents.

Recombinant murine IL-18 (SEQ ID NO:2) has demonstrated pre-clinical anti-tumor activity through a variety of mechanisms, including activation of CD4⁺, CD8⁺, and NK cells as well as the Fas/FasL pathway and cytokines/chemokines such as INFγ, GM-CSF, IP-10, MCP-1, and infiltration of effector cells in tumors and induction of immunological memory. The benefits of IL-18, such as induction of cytolytic T cells, expansion of activated NK cells, and cells that play key role in antibody-dependent cellular cytoxicity (ADCC) has been demonstrated in our pre-clinical models.

The below examples investigated whether the combination of IL-18 with other clinically relevant cancer treatments would result in enhanced anti-tumor activity that was superior to human IL-18 monotherapy alone. This application exemplifies five examples of IL-18 combination therapies: (1) the combination of IL-18 and RITUXAN^{®} in the human B cell lymphoma xenograft model; (2) the combination of IL-18 and doxorubicin in the syngeneic T-cell lymphoma model; (3) the combination of IL-18 and HERCEPTIN^{®} in a murine plasmocytoma model; and (4) the combination of IL- 18 and 5-FU in a murine colon tumor model; and (5) the combination of IL-18 and pazopanib, an inhibitor of VEGFR, PDGFR, and c-kit tyrosine kinases, in a murine model of renal carcinoma. All of these combinations offered benefits over the monotherapy with, IL-18, RITUYAN^{®}, doxorubicin, HERCEPTIN^{®}, 5-FU, or pazopanib alone.

Combination with monoclonal antibodies offers a potential for enhancement of ADCC mechanism of tumor cell killing. The pre-clinical data disclosed in this application support this mechanism, and showed enhancement of anti-tumor activity of RITUXAN^{®} in combination with mIL-18 (SEQ ID NO:2). Several mechanisms may contribute to the efficacy of RITUXAN^{®}; however, accumulating evidence suggests that ADCC plays a dominant role in elimination of tumor cells after administration of RITUXAN^{®} . ADCC is triggered when the constant (Fc) region of an antibody binds to Fc receptors on the surface of effector cells, such as natural killer (NK) cells or cells of monocyte/macrophage lineage. In a murine model of human B cell lymphoma, the efficacy of RITUXAN^{®} was abrogated in mice lacking activating Fc receptors. Cartron, et al., Blood 99: 754-758 (2002); Dall'Ozzo, et al. Cancer Res 64: 4664-4669 (2004); Koene, et al. Blood 90: 1109-1114 (1997); Weng, et al. J Clin Oncol 21: 3940-3947 (2003). Thus, Fc receptor-bearing effector cells were critical for the efficacy of RITUXAN^{®}. CD16 (FcγRIIIA) is an important Fc receptor in humans, which is expressed by NK cells and macrophages. *Id.* The data in Example 1 support the hypothesis that NK cell-mediated ADCC is important for the effectiveness of RITUXAN^{®} therapy in patients with lymphoma.

One promising strategy for improving the efficacy of RITUXAN^{®} is to administer cytokines, such as IL-18, that can cause the expansion and/or activation of Fc receptor-bearing effector cells, including NK cells and cells of monocyte/macrophage lineage. The pre-clinical mouse tumor model studies with IL-18 in combination with RITUXAN^{®} in Example 1 showed benefit over the monotherapies. In this model, the full benefit of IL-18 could not be tested, since the model required human xenograft in the SCID immuno-compromised mouse that has only NK functional cells. However, the data in Example 1 support that expansion of these ADCC NK effector cells showed benefit in the IL-18 and RITUXAN^{®} combo. RITUXAN^{®} as active as monotherapy at the highest dose tested. However, similar levels of activity could be seen when lower doses of RITUXAN^{®} were used in combination with mIL-18 (SEQ ID NO:2), indicating both that the model was sensitive to the mechanism of RITUXAN^{®}, and that the response could be enhanced by IL-18. It is believed that combinations of IL-18 with other monoclonal antibodies against antigens, such as CD22, CD19, HER2, HER3, EGFR (Erbitux), IGF-1R, IGF-1R, AXL-1, FGFR, integrin receptors, CEA, CD44 and VEGFR, and other anti-angiogenic agents would show the same synergistic effects. In fact, it is futher envisiaged that similar combinations of IL-18 with other monoclonal antibodies against antigens are found on the surface of tumor cells that expresses a receptor to which a monoclonal antibody is generated, would work the same way. Ideally, such a receptor would bind NK cells, monocytes, macrophages, B-cells, T-cells, and any other cells that contain Fc receptors and participate in ADCC effector activity.

The data in Examples 1 and 2 suggest that the combination of anti-cancer agents with IL-18 may show clinical benefit, since these combinations provide two different mechanisms of action: one is a direct effect on the tumor cells, while IL-18 is capable of augmenting a patient's immune cells. These two mechanisms could complement each other, and potentially resulting in long-lasting, superior anti-tumor activity, due to IL-18's capability to generate immunological memory. Overall, Examples 1 and 2 demonstrate that the combination of IL-18 with anti-tumor agents, either monoclonal antibodies or chemotherapeutics, results in synergy and superior activity.

Combinations of IL-18 with the chemotherapeutic agent, doxorubicin, showed superior anti-tumor activity over either IL-18, or doxorubicin alone. Notably, Example 2 shows that the combination of IL-18 with doxorubicin did not destroy the activated immune cells that are expanded in response to IL-18 treatment. Surprisingly, to the contrary, Example 2 demonstrates that the combination augments the activated T and NK cells, and maintains their cytolytic function.

Example 3 is a Phase I clinical protocol that is currently underway to evaluate the safety and biological activity of IL-18 in combination with Rituximab in patients with CD20+ B cell non-Hodgkin's lymphoma (NHL). This study uses a standard treatment regimen of Rituximab in combination with rising doses of IL-18 to identify a dose that is safe and tolerable and gives a maximum biological effect, as demonstrated by selected biomarkers (e.g., activated NK cells). The dose selected from this study will be used in a future Phase II study evaluating the efficacy of the IL-18/Rituximab combination in patients with relapsed follicular lymphoma. Given the good safety and tolerability profile of IL-18 when administered as monotherapy to patients with metastatic melanoma, it is not anticipated that the maximum tolerated dose (MTD) of the combination will be reached in the current study; however, this study is designed to define the MTD, if dose-limiting toxicities are identified in patients with non-Hodgkin's lymphoma.

Example 4 provides an analysis and data for the combination therapy of human IL-18 with HERCEPTIN^{®} on the growth of murine plasmocytoma (transfected MoPC315 cells with ErbB2 (HER2)). A detailed analysis of the data revealed that the combinational therapy with IL-18 and HERCEPTIN^{®} surpasses the monotherapy with HERCEPTIN^{®} alone. Based upon these data, it is believed that human IL-18 will be an effective therapeutic combination with other antibodies of antigens that are expressed on tumor cells.

Example 5 evaluates the efficacy of IL-18 combination therapy with 5-fluorouracil (5-FU), as compared to monotherapy with 5-FU, or mIL-18 alone. 5-FU is a pyrimidine analog, currently used in clinics as one of the first-line chemotherapeutics for treatment of colorectal and pancreatic cancer. This chemotherapeutic, however, has multiple serious side-effects, and a possibility to lower its dose using a combination therapy with other agents is desirable. This study was performed in a well established syngeneic subcutaneous model of murine colon carcinoma, Colo 26, in BALB/c mice. A detailed analysis of the tumor volume data in Example 5 revealed that the combinational therapy with 10 µg of IL-18 and 75 µg of 5-FU is the only treatment group with the significant effect on tumor growth, as compared to the control group. This means that the combination therapy (75 µg /10 µg) surpassed the monotherapy groups with 5-FU alone, or with mIL-18 alone, because monotherapy did not show a therapeutic effect better than a control. Other chemotherapeutic agents in combination with IL-18, such as doxil, topotecan, DNA-altering drugs (e.g., carboplatin), antimetabolites (e.g., gemcitabine), drugs that prevent cell division (e.g., vincristine) and anti-angiogenic agents (e.g., pazopanib).

Example 6 provides a study of the efficacy of combination therapy with IL-18 and pazopanib (GW786034), an inhibitor of VEGFR and PDCFR and c-kit tyrosine kinases, in a mouse renal cell carcinoma model. The c-Kit receptor belongs to type III tyrosine kinase receptor, which consists of an extracellular ligand binding domain and an intracellular kinase domain. The c-Kit receptor is expressed in a wide variety of normal and neoplastic tissues. These data show that combination of pazopanib with IL-18 results in anti-tumor activity (synergy) that is statistically significant when compared to each monotherapy alone.

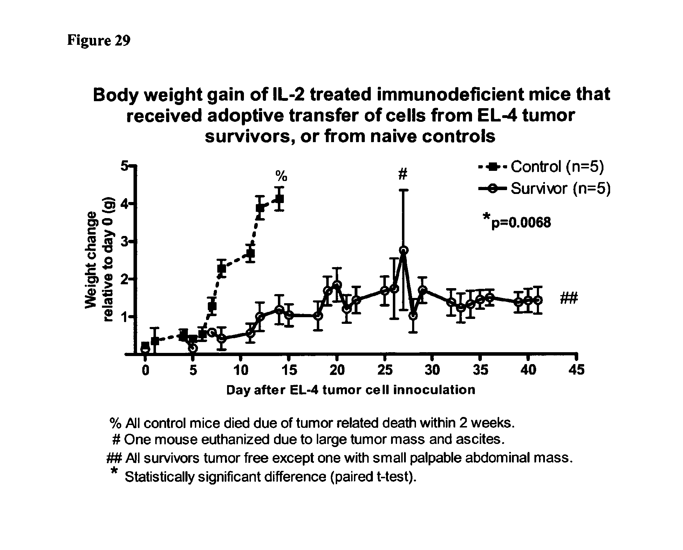

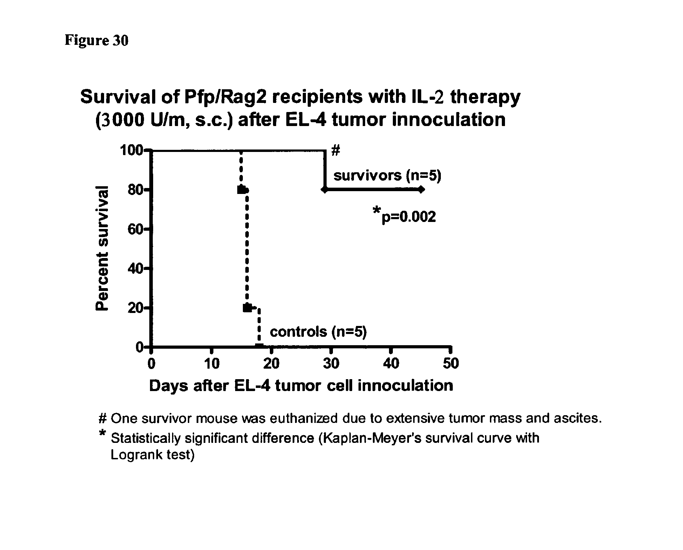

Example 7 is a study that addresses the role of IL-18 as an inducer of memory that would result in long- term survival and prevention of tumor relapse. This example tests the efficacy in a EL-4 tumor model, where mice were treated by combination of murine IL-18 (SEQ ID NO:2) and doxorubicin. The EL-4 recipient mice that received survivor lymphatic cells survived significantly longer than control mice that received lymphatic cells from normal naive donors. The data imply that the adoptive transfer from survivor mice had a protective effect on the EL-4 tumor recipients. These data offer an indirect demonstration of memory T cells in the EL-4 tumor survivors (Figure 29 and Figure 30). This is an important finding that could make combination of any chemotherapeutic agent or mAb with IL-18 a superior cancer treatment to any monotherapy. Induction of memory T-cells that could recognize tumor as "foreign" and prevent relapse would be highly beneficial, and IL-18 with its good safety profile, a drug for any potential combination therapy.

Human IL-18 polypeptides are disclosed in EP 0692536A2, EP 0712931A2, EP0767178A1, and WO 97/2441. The amino acid sequence of native human IL-18 ("hIL-18) is set forth in SEQ ID NO:1. Human IL-18 polypeptides are interferon-γ-inducing polypeptides. They play a primary role in the induction of cell-mediated immunity, including induction of interferon-γ production by T cells and splenocytes, enhancement of the killing activity of NK cells, and promotion of the differentiation of naive CD4+ T cells into Th1 cells.

Polypeptides of the present invention can be recovered and purified from recombinant cell cultures by well known methods, including ammonium sulfate or ethanol precipitation, acid extraction, anion or cation exchange chromatography, phosphocellulose chromatography, hydrophobic interaction chromatography, affinity chromatography, hydroxylapatite chromatography, lectin chromatography, and high performance liquid chromatography. Well known techniques for refolding proteins may be employed to regenerate active conformation when the polypeptide is denatured during intracellular synthesis, isolation and/or purification. Methods to purify and produce active human IL-18 are set forth in WO 01/098455.

The present invention also provides pharmaceutical compositions comprising human IL-18 polypeptides (SEQ ID NO:1) and combinations thereof. Such compositions comprise a therapeutically effective amount of a compound, and may further comprise a pharmaceutically acceptable carrier, diluent, or excipient. Such pharmaceutical carriers can be sterile liquids, such as water and oils, including those of petroleum, animal, vegetable or synthetic origin, such as peanut oil, soybean oil, mineral oil, sesame oil, *etc.* Water can be used as a carrier when the pharmaceutical composition is administered intravenously. Saline solutions and aqueous dextrose and glycerol solutions can also be employed as liquid carriers, for example, for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations, and the like. The composition can be formulated as a suppository, with traditional binders and carriers, such as triglycerides. Oral formulation can include standard carriers, such as pharmaceutical grades of mannitol, lactose, starch, magnesium stearate, sodium saccharine, cellulose, magnesium carbonate, *etc.* Examples of suitable pharmaceutical carriers are described in REMINGTON'S PHARMACEUTICAL SCIENCES by E. W. Martin. Such compositions will contain a therapeutically effective amount of the compound, often in purified form, together with a suitable amount of carrier so as to provide the form for proper administration to the patient. The formulation should suit the mode of administration.

In one embodiment of the invention, the composition is formulated in accordance with routine procedures as a pharmaceutical composition adapted for intravenous administration to human beings. Typically, compositions for intravenous administration are solutions in sterile isotonic aqueous buffer. Where suitable, the composition may also include a solubilizing agent and a local anesthetic, such as lignocaine, to ease pain at the site of the injection. Generally, the ingredients are supplied either separately or mixed together in unit dosage form, for example, as a dry lyophilized powder, or water-free concentrate, in a hermetically sealed container, such as an ampoule or sachette, indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

Accordingly, the polypeptide may be used in the manufacture of a medicament. Pharmaceutical compositions of the invention may be formulated as solutions or as lyophilized powders for parenteral administration. Powders may be reconstituted by addition of a suitable diluent or other pharmaceutically acceptable carrier prior to use. The liquid formulation may be a buffered, isotonic, aqueous solution. Examples of suitable diluents are normal isotonic saline solution, standard 5% dextrose in water or buffered sodium or ammonium acetate solution. Such a formulation is especially suitable for parenteral administration, but may also be used for oral administration or contained in a metered dose inhaler or nebulizer for insufflation. It may be desirable to add excipients, such as polyvinylpyrrolidone, gelatin, hydroxy cellulose, acacia, polyethylene glycol, mannitol, sodium chloride, or sodium citrate, to such pharmaceutical compositions.

Alternately, the polypeptide may be encapsulated, tableted or prepared in an emulsion or syrup for oral administration. Pharmaceutically acceptable solid or liquid carriers may be added to enhance or stabilize the composition, or to facilitate preparation of the composition. Solid carriers include starch, lactose, calcium sulfate dihydrate, terra alba, magnesium stearate or stearic acid, talc, pectin, acacia, agar, or gelatin. Liquid carriers include syrup, peanut oil, olive oil, saline, and water. The carrier may also include a sustained release material, such as glyceryl monostearate or glyceryl distearate, alone or with a wax. The amount of solid carrier varies but, will be between about 20 mg to about 1 g per dosage unit. The pharmaceutical preparations are made following the conventional techniques of pharmacy involving milling, mixing, granulating, and compressing, when suitable, for tablet forms; or milling, mixing and filling for hard gelatin capsule forms. When a liquid carrier is used, the preparation will be in the form of a syrup, elixir, emulsion, or an aqueous, or non-aqueous suspension. Such a liquid formulation may be administered directly by mouth (p.o.) or filled into a soft gelatin capsule.

Human IL-18 polypeptides may be prepared as pharmaceutical compositions containing an effective amount the polypeptide as an active ingredient in a pharmaceutically acceptable carrier. In the compositions of the invention, an aqueous suspension or solution containing the polypeptide, buffered at physiological pH, in a form ready for injection may be employed. The compositions for parenteral administration will commonly comprise a solution of the polypeptide of the invention or a cocktail thereof dissolved in a pharmaceutically acceptable carrier, such as an aqueous carrier. A variety of aqueous carriers may be employed, *e*.*g*., 0.4% saline, 0.3% glycine, and the like. These solutions are sterile and generally free of particulate matter. These solutions may be sterilized by conventional, well known sterilization techniques (e.g., filtration). The compositions may contain pharmaceutically acceptable auxiliary substances as required to approximate physiological conditions such as pH adjusting and buffering agents, *etc.* The concentration of the polypeptide of the invention in such pharmaceutical formulation can vary widely, *i.e.,* from less than about 0.5%, usually at or at least about 1% to as much as 15 or 20% by weight and will be selected primarily based on fluid volumes, viscosities, *etc.,* according to the particular mode of administration selected.

Thus, a pharmaceutical composition of the invention for intramuscular injection could be prepared to contain 1 mL sterile buffered water, and between about 1 ng to about 100 mg, *e.g,*. about 50 ng to about 30 mg, or from about 5 mg to about 25 mg, of a polypeptide of the invention. Similarly, a pharmaceutical composition of the invention for intravenous infusion could be made up to contain about 250 mL of sterile Ringer's solution, and about 1 mg to about 30 mg, or from about 5 mg to about 25 mg of a polypeptide of the invention. Actual methods for preparing parenterally administrable compositions are well known or will be apparent to those skilled in the art and are described in more detail in, for example, REMINGTON'S PHARMACEUTICAL SCIENCE, 15th ed., Mack Publishing Company, Easton, Pennsylvania.

The polypeptides of the invention, when prepared in a pharmaceutical preparation, may be present in unit dose forms. The appropriate therapeutically effective dose can be determined readily by those of skill in the art. Such a dose may, if suitable, be repeated at appropriate time intervals selected as appropriate by a physician during the response period. In addition, *in vitro* assays may optionally be employed to help identify optimal dosage ranges. The precise dose to be employed in the formulation will also depend upon the route of administration, and the seriousness of the disease or disorder, and should be decided according to the judgment of the practitioner and each patient's circumstances. Effective doses may be extrapolated from dose-response curves derived from *in vitro* or animal model test systems.

For polypeptides, the dosage administered to a patient is typically 0.1 mg/kg to 100 mg/kg of the patient's body weight. The dosage administered to a patient may be between 0.1 mg/kg and 20 mg/kg of the patient's body weight, or alternatively, 1 mg/kg to 10 mg/kg of the patient's body weight. Generally, human polypeptides have a longer half-life within the human body than polypeptides from other species, due to the immune response to the foreign polypeptides. Thus, lower dosages of human polypeptides and less frequent administration is often possible. Further, the dosage and frequency of administration of polypeptides of the invention may be reduced by enhancing uptake and tissue penetration (e.g., into the brain) of the polypeptides by modifications such as, for example, lipidation.

The invention also provides a pharmaceutical pack or kit comprising one or more containers filled with one or more of the ingredients of the pharmaceutical compositions of the invention. Optionally associated with such container(s) can be a notice in the form prescribed by a governmental agency regulating the manufacture, use or sale of pharmaceuticals or biological products, which notice reflects approval by the agency of manufacture, use or sale for human administration. In another embodiment of the invention, a kit can be provided with the appropriate number of containers required to fulfill the dosage requirements for treatment of a particular indication.

In another embodiment, the compound or composition can be delivered in a vesicle, in particular a liposome (see Langer, Science 249:1527-1533 (1990); Treat, et al., in LIPOSOMES IN THE THERAPY OF INFECTIOUS DISEASE AND CANCER, Lopez-Berestein and Fidler (eds.), Liss, New York, pp. 353-365 (1989); Lopez-Berestein, ibid., pp. 317-327; *see generally ibid.*)*.*

In yet another embodiment, the compound or composition can be delivered in a controlled release system. In one embodiment, a pump may be used (*see* Langer, *supra*; Sefton, CRC Crit. Ref Biomed. Eng. 14:201 (1987); Buchwald, et al., Surgery 88:507 (1980); Saudek, et al., N. Engl. J. Med. 321:574 (1989)). In another embodiment, polymeric materials can be used (*see* MEDICAL APPLICATIONS OF CONTROLLED RELEASE, Langer and Wise (eds.), CRC Pres., Boca Raton, Fla. (1974); CONTROLLED DRUG BIOAVAILABILITY, DRUG PRODUCT DESIGN AND PERFORMANCE, Smolen and Ball (eds.), Wiley, New York (1984); Ranger, et al., J., Macromol. Sci. Rev. Macromol. Chem. 23:61 (1983); *see also* Levy, et al., Science 228:190 (1985); During, et al., Ann. Neurol. 25:351 (1989); Howard, et al., J. Neurosurg. 71:105 (1989)). In yet another embodiment, a controlled release system can be placed in proximity of the therapeutic target, i.e., the brain, thus requiring only a fraction of the systemic dose (*see, e.g.,* Goodson, in MEDICAL APPLICATIONS OF CONTROLLED RELEASE, supra, vol. 2, pp. 115-138 (1984)). Other controlled release systems are discussed in the review by Langer (Science 249:1527-1533 (1990)).

Human IL-18 polypeptides (SEQ ID NO:1) may be administered by any appropriate internal route, and may be repeated as needed, e.g., as frequently as one to three times daily for between 1 day to about three weeks to once per week or once biweekly. Alternatively, the peptide may be altered to reduce charge density and thus allow oral bioavailability. The dose and duration of treatment relates to the relative duration of the molecules of the present invention in the human circulation, and can be adjusted by one of skill in the art, depending upon the condition being treated and the general health of the patient.

The invention provides methods of treatment, inhibition and prophylaxis by administration to a human patient an effective amount of a compound or pharmaceutical composition of the invention comprising human IL-18 polypeptide (SEQ ID NO:1). In one embodiment of the invention, the compound is substantially purified (*e*.*g*., substantially free from substances that limit its effect or produce undesired side-effects). Formulations and methods of administration can be employed when the compound comprises a polypeptide as described above; additional appropriate formulations and routes of administration can be selected from among those described herein below.

Various delivery systems are known and can be used to administer a compound of the invention, *e*.*g*., encapsulation in liposomes, microparticles, microcapsules, recombinant cells capable of expressing the compound, receptor-mediated endocytosis (*see, e.g.,* Wu, et al., J. Biol. Chem. 262:4429-4432 (1987)), construction of a nucleic acid as part of a retroviral or other vector, *etc.* Methods of introduction include, but are not limited to, intradermal, intramuscular, intraperitoneal, intravenous, subcutaneous, intranasal, epidural, and oral routes. The compounds or compositions may be administered by any convenient route, for example by infusion or bolus injection, by absorption through epithelial or mucocutaneous linings (e.g., oral mucosa, rectal and intestinal mucosa, *etc.*) and may be administered together with other biologically active agents. Administration can be systemic or local. In addition, it may be desirable to introduce the pharmaceutical compounds or compositions of the invention into the central nervous system by any suitable route, including intraventricular and intrathecal injection; intraventricular injection may be facilitated by an intraventricular catheter, for example, attached to a reservoir, such as an Ommaya reservoir. Pulmonary administration can also be employed, e.g., by use of an inhaler or nebulizer, and formulation with an aerosolizing agent.

The present invention may be embodied in other specific forms, without departing from the spirit or essential attributes thereof, and, accordingly, reference should be made to the appended claims, rather than to the foregoing specification or following examples, as indicating the scope of the invention.

### Glossary

The following definitions are provided to facilitate understanding of certain terms used frequently hereinbefore.

"Antibody-Dependent Cell-Mediated Cytotoxicity (ADCC)" and "Antibody-Dependent Cell-Mediated Cytotoxicity (ADCC) effector function", as used herein, both pertain to a mechanism of cell-mediated immunity, whereby an effector cell of the immune system actively lyses a target cell that has been bound by specific antibodies. ADCC is one of the mechanisms through which antibodies, as part of the humoral immune response, can act to limit and contain infection. Classical ADCC is mediated by natural killer (NK) cells, but an alternate ADCC is used by eosinophils to kill certain parasitic worms known as helminths. ADCC is part of the adaptive immune response due to its dependence on a prior antibody response.

The typical ADCC involves activation ofNK cells and is dependent upon the recognition of antibody-coated infected cells by Fc receptors on the surface of the NK cell. The Fc receptors recognize the Fc (constant) portion of antibodies such as IgG, which bind to the surface of a pathogen-infected target cell. The Fc receptor that exists on the surface ofNK Cell is called CD16 or FcγRIII. Once bound to the Fc receptor of IgG the Natural Killer cell releases cytokines such as IFN-γ, and cytotoxic granules, such as perforin and granzyme, that enter the target cell and promote cell death by triggering apoptosis. This ADCC effector function is similar to, but independent of, responses by cytotoxic T cells (CTLs).

As used herein, the term, "carrier", refers to a diluent, adjuvant, excipient, or vehicle with which the therapeutic is administered.

The term, "complete response", as used herein, means the disappearance of all signs of cancer in response to treatment. Those of skill in the art also call a "complete response" a "complete remission". In the models employed in the below examples, an animal achieving a "complete response" means that measurable tumors regressed to stage that could not be measured. In other words, it means that animals were "cured" and appeared healthy.

"Isolated" means altered "by the hand of man" from its natural state, i. e., if it occurs in nature, it has been changed or removed from its original environment, or both. For example, a polynucleotide or a polypeptide naturally present in a living organism is not "isolated," but the same polynucleotide or polypeptide separated from at least one of its coexisting cellular materials of its natural state is "isolated", as the term is employed herein. Moreover, a polynucleotide or polypeptide that is introduced into an organism by transformation, genetic manipulation or by any other recombinant method is "isolated" even if it is still present in said organism, which organism may be living or non-living.

As used herein, the term, "pharmaceutical", includes veterinary applications of the invention. The term, "therapeutically effective amount", refers to that amount of therapeutic agent, which is useful for alleviating a selected condition.

As used herein, the term, "pharmaceutically acceptable", means approved by a regulatory agency of the Federal or a state government or listed in the U. S. Pharmacopeia or other generally recognized pharmacopeia for use in animals, and more particularly in humans.

"Polypeptide" refers to any polypeptide comprising two or more amino acids joined to each other by peptide bonds or modified peptide bonds, i.e., peptide isosteres. "Polypeptide" refers to both short chains, commonly referred to as peptides, oligopeptides or oligomers, and to longer chains, generally referred to as proteins. Polypeptides may contain amino acids other than the 20 gene-encoded amino acids. "Polypeptides" include amino acid sequences modified either by natural processes, such as post-translational processing, or by chemical modification techniques that are well known in the art. Such modifications are well described in basic texts and in more detailed monographs, as well as in a voluminous research literature. Modifications may occur anywhere in a polypeptide, including the peptide backbone, the amino acid side-chains and the amino or carboxyl termini. It will be appreciated that the same type of modification may be present to the same or varying degrees at several sites in a given polypeptide. Also, a given polypeptide may contain many types of modifications. Polypeptides may be branched as a result of ubiquitination, and they may be cyclic, with or without branching. Cyclic, branched and branched cyclic polypeptides may result from post-translation natural processes or may be made by synthetic methods. Modifications include acetylation, acylation, ADP-ribosylation, amidation, biotinylation, covalent attachment of flavin, covalent attachment of a heme moiety, covalent attachment of a nucleotide or nucleotide derivative, covalent attachment of a lipid or lipid derivative, covalent attachment ofphosphotidylinositol, cross-linking, cyclization, disulfide bond formation, demethylation, formation of covalent cross-links, formation of cysteine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins, such as arginylation, and ubiquitination (*see*, for instance, PROTEINS - STRUCTURE AND MOLECULAR PROPERTIES, 2nd Ed., T. E. Creighton, W. H. Freeman and Company, New York, 1993; Wold, F., Post-translational Protein Modifications: Perspectives and Prospects, 1-12, in POST-TRANSLATIONAL COVALENT MODIFICATION OF PROTEINS, B. C. Johnson, Ed., Academic Press, New York, 1983; Seifter et al., Meth Enzymol, 182, 626-646, 1990; Rattan, et al., Ann. NY Acad. Sci., 663: 48-62 (1992)).

As used herein, the term "surviving animal(s)", means the animal(s) that did not die of spontaneous tumor related death, or were not euthanized due to tumor volume reaching the pre-determined in-humanely enormous size, or were not euthanized due to drug toxicity-related reason.

### Biological Methods/Examples

### Example 1: Experimental Protocol for IL-18 combination therapy with RITUXAN^{®} in a murine human B-cell lymphoma model

Human IL-18 (SEQ ID NO:1) is a recombinant mature form of human interleukin-18, expressed in a non-pathogenic strain of *Escherichia coli.* IL-18 is a non-glycosylated monomer of 18Kd with a primary structure most closely related to IL-β and the IL-1 trefoil sub-family. Murine and human IL-18 cDNA encode a precursor protein consisting of 192 and 193 amino acids (SEQ ID NOs: 2 and 1, respectively). Pro-IL-18 requires processing by caspases into bioactive mature protein (157 amino acids) in order to mediate its biological activity. The homology between human and murine IL-18 is 65%. In the pre-clinical studies outlined below, murine IL-18 (SEQ ID NO:2) was used, in order to provide an *in vivo* syngeneic system, where the full immunological potential of IL-18 could be analyzed.

The study was performed in outbred female homozygous SCID mice (ICR-*Prkdc^{Scid})* that lack both T and B cells. The advantage of using the outbred stock over the inbred strain is that the outbred ICR SCID strain does not exhibit leakiness (even in 10-12 month old mice).

Mice were injected with human Ramos B-cell lymphoma line that was originally derived from a 3-year-old patient with Burkitt's lymphoma (ATCC catalogue, CRL 1596). The tumor 1:10 homogenate was inoculated into 6-8 week old mice at the dose 0.5 ml per mouse. The tumor volume was measured 2-3 times a week, and mice were randomly distributed into the treatment groups so that the groups had equal distribution of tumor volumes. The therapy was initiated when the median tumor volume per group reached 80 - 150 mm³ (at day 12 post tumor inoculation). In addition, those mice that grew a tumor with a volume outside of the set limits were excluded from the study.

In the first study, the treatment groups (n=6) included a control group (no therapy), three RITUXAN^{®} LV. monotherapy groups (12.5, 25, and 50µg/mouse BIW, respectively), a mIL-18 S.C. monotherapy group (100 µg/mouse q.d.)., and three combinational therapy groups that each received 100 µg/mouse IL-18 S.C. q.d. plus 12.5, 25, or 50 µg/mouse RITUXAN^{®} I.V., respectively.

In the second study, the dosing consisted of mIL-18 (SEQ ID NO:2) at 100 µg/mouse on an SID schedule, and RITUXAN^{®} at 25 and 12.5µg on qd4/3 schedule. The number of animals was increased to n=12, in order to have a better window to measure statistical significance. Tumor volume was measured using the viener calipers two to three times a week.

The combinational therapy with IL-18 and RITUXAN^{®} in the human B-cell lymphoma model offers a benefit over the monotherapy with either IL-18, or RITUXAN^{®} alone. Two experiments, detailed below, show a statistically significant benefit of the combination therapy in this model.

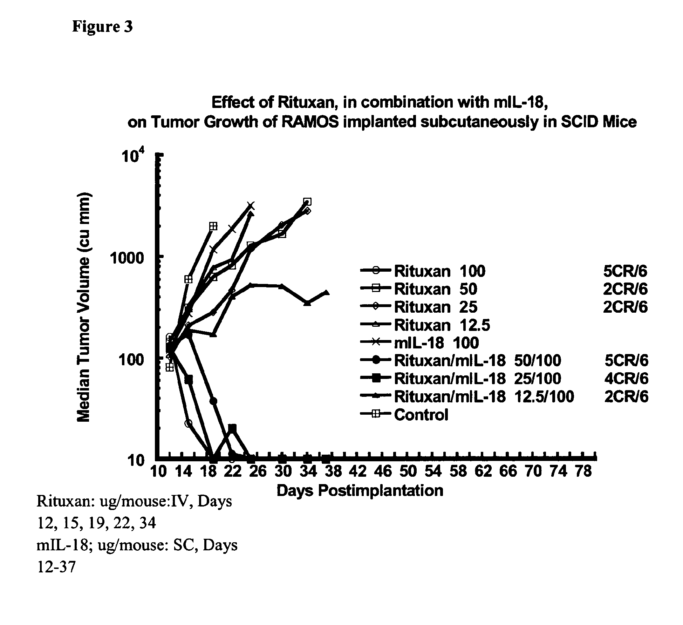

In the first experiment, captured in Figure 3, the high dose of RITUXAN^{®} (100µg/dose) showed strong anti-tumor activity as a single agent therapy, while at lower dose (12.5g/dose), RITUXAN^{®} had no activity. Murine IL-18 (SEQ ID NO:2) had no activity as a single agent (100µg/dose). However, when combined with a lower dose of RITUXAN^{®}, mIL-18 (SEQ ID NO:2) showed additive/synergistic activity (12.5µg/dose of RITUXAN^{®} combined with 100µg of mIL-18 (SEQ ID NO:2).

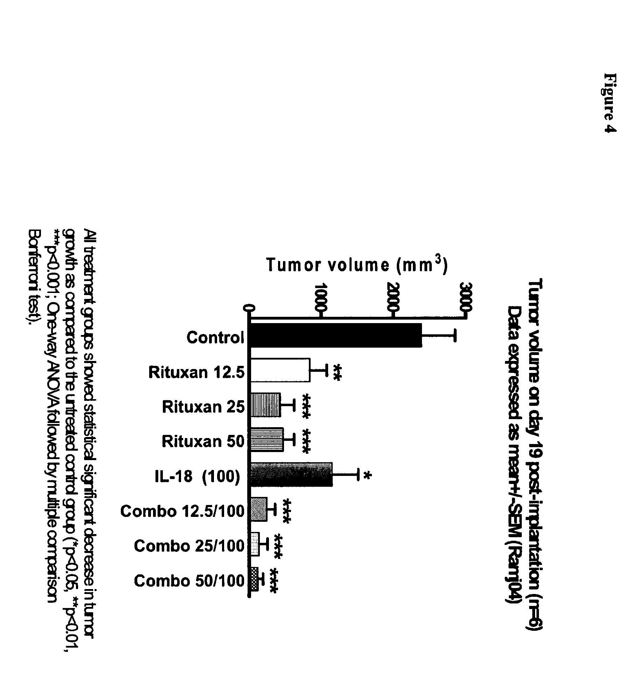

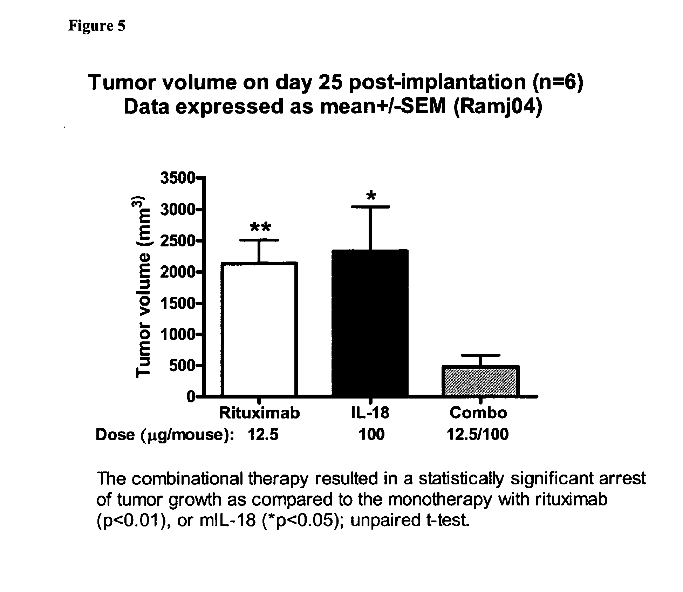

The statistical significance is demonstrated below in Figures 4 and 5, when the data are graphed and analysed using GraphPad Prism^{®}. In the first of these graphs, Figure 4, the tumor volumes are compared on day 19 post-implantation. The statistical analysis showed a significant decrease of tumor growth in all treatment groups as compared to the untreated control group (*p<0.05, **p<0.01, ***p<0.001). The second graph, Figure 5, shows that the combination therapy was more effective (statistically significant, *p<0.05, **p<0.01) than monotherapies alone.

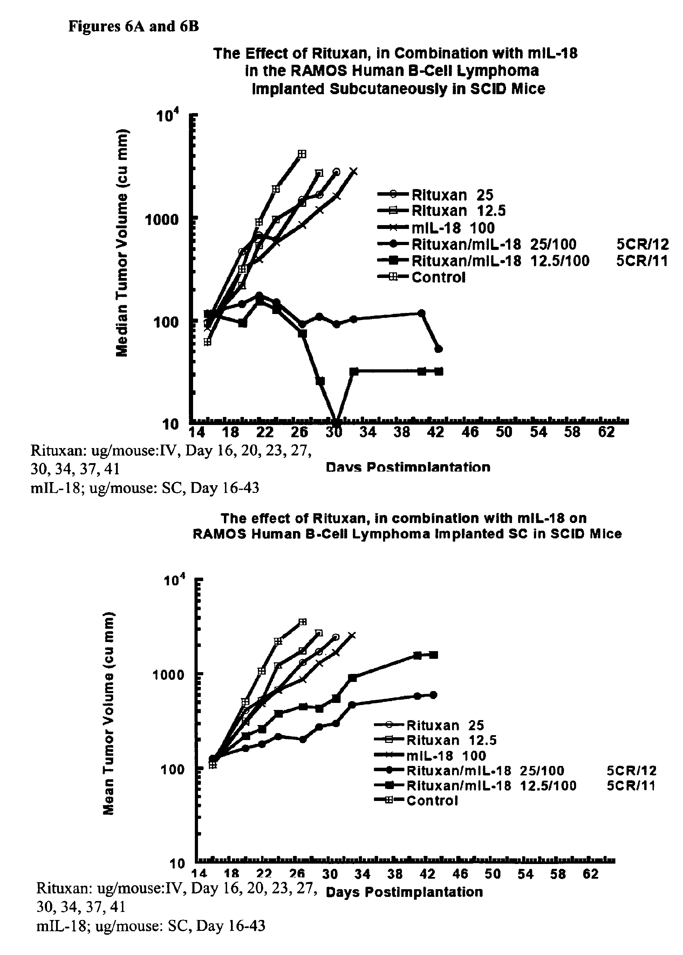

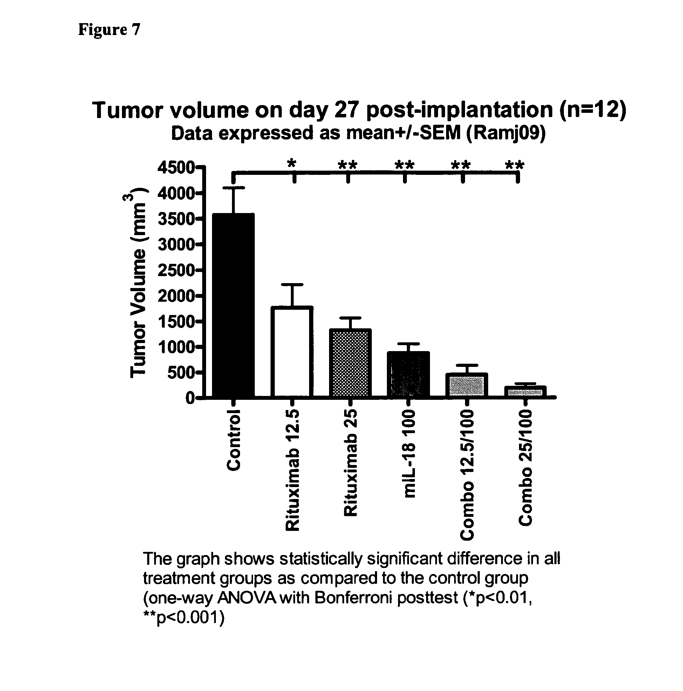

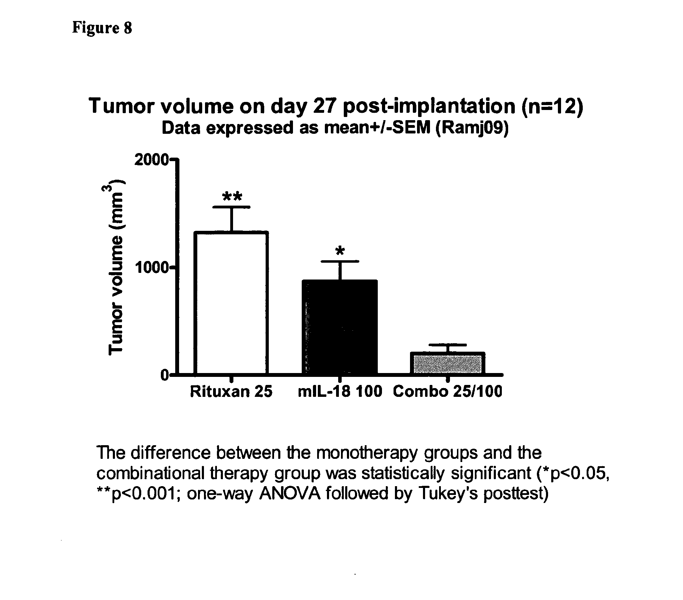

In the second experiment, increasing the number of animals (n=12) provided better statistical significance of the additive/synergistic anti-tumor activity in response to combination therapy. The graphs in Figures 6A and 6B represent median and mean tumor growth volume. The study was analyzed at day 27 post-tumor implantation. This study is on-going, and will be terminated when median tumor volume will reach 2000 cu mm (ACUC protocol). However, the data analysis on day 27 post-implantation, Figures 7 and 8, demonstrates that there is a statistically significant decrease of tumor volume in mice treated with combinational therapy (25/100 µg/mouse), as compared to the RITUXAN^{®} alone (25 µg/mouse) or mIL-18 (SEQ ID NO:2) monotherapy alone (100 µg/mouse).

This pre-clinical data demonstrates that the combination ofIL-18 and Rituximab results in synergistic anti-tumor activity. Rituximab was active as monotherapy at the highest dose tested. However, similar levels of activity could be seen when lower doses of Rituximab were used in combination with murine IL-18 ("mIL-18"), indicating that the model was sensitive to Rituximab and that the response could be enhanced by IL-18. Murine IL-18 enhanced the activity of Rituximab, presumably by augmenting ADCC activity in NK cells. Since SCID mice lack both B and T-cell responses, IL-18 is augmenting anti-tumor responses through NK cell activation.

In addition, the administration of IL-18 to non-human primates produced activation of NK cells and monocytes *in vivo* and led to an up-regulation of Fc receptors (FcγRI) on monocytes. Herzyk, et al., Cytokine 20:38-48 (2002). Synergistic anti-tumor activity has also been observed when IL-18 is used in combination with HERCEPTIN™ in a SCID mouse model, supporting the hypothesis that IL-18 is increasing ADCC activity through NK cell activation.

### Example 2: Experimental Protocol for IL-18 combination with doxorubicin in EL-4 T cell lymphoma,

Studies were performed in female C57/BL/6 mice. As a general protocol, C57/BL mice were injected I.P. with 0.2 cc of stock EL-4 cells. EL-4 murine T-lymphoma cells were expanded in RPMI w/10%FCS. All animals were randomized to six or seven mice per study group with food and water ad libitum. EL-4 cells were harvested on day 0, counted and implanted I.P. with 5x10⁵ EL-4 lymphoma cells. Animals were randomized to treatment groups of 6/7 animals on Day 3. Doxorubicin was administered IV on Days 3 &10, pos-implantation. mIL-18 (SEQ ID NO:2) was administered S.C. on Days 3-16. The animals were observed daily for toxicity and mortality.

All animals tolerated dosing schedule and levels well by gross observation. On day 16, all dosing was terminated, and median vehicle death occurred on day 17.5. All vehicle mice expired between days 16-18 post-implantation. Increase in lifespan was calculated by study group/vehicle group ― 1 x100%. For mIL-18 (SEQ ID NO:2) combination therapy with doxorubicin in EL-4 T cell lymphoma, the data were analyzed with respect to median survival time and increase in lifespan.

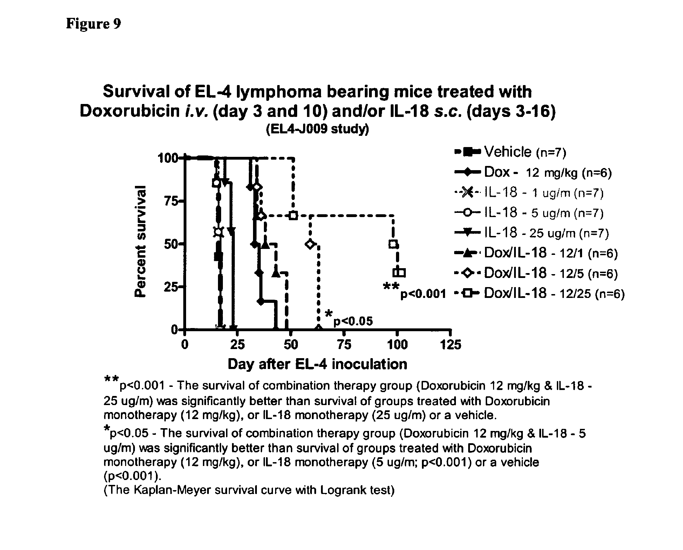

The prolongation of life span in response to combinational therapy with mIL-18 (SEQ ID NO:2) and doxorubicin was assessed in the syngeneic tumor model of female C57B1/6 mice bearing EL-4 T-cell lymphoma. The benefit of combination therapy over the monotherapy with either mIL-18 (SEQ ID NO:2) alone, or doxorubicin alone, was demonstrated in several experiments, detailed below. The example of anti-tumor activity and prolongation of life span is demonstrated in Figure 9. As described below, when vehicle animals expired (on day 16) all dosing was terminated. Median vehicle death occurred on day 17.5, and all vehicle mice expired between days 16-18 post-implantation.

These results show that the combination of doxorubicin and mIL-18 (SEQ ID NO:2) in EL-4 T-cell lymphoma results in synergistic anti-tumor activity with increased survival. The doxorubicin monotherapy showed minimal increase in lifespan at 12 mg/kg dose. IL-18 monotherapy at doses of 1, 5 and 25ug/dose did not show any increase in lifespan. When 12mg/kg of doxorubicin was combined with 25 µg/dose of IL-18, there was a shift towards increased survival and increased cure. When these animals were re-challenged with tumor, they showed protection.

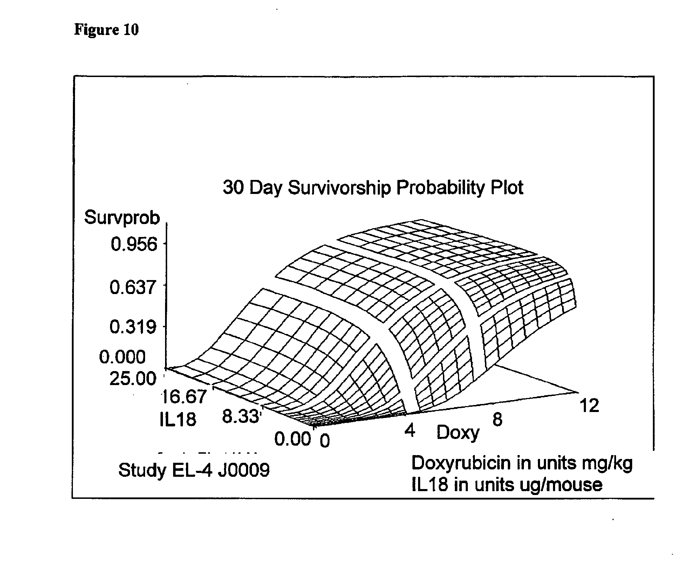

The inventors then examined the predictability of surviving for a combination therapy of IL-18 and doxorubicin was then examined, recognizing that it would be advantageous to identify the best dose for both reagents that would result in synergistic anti-tumor activity. The survivor probability plot of the data shown in Figure 9 is shown in Figure 10.

For this analysis, we used data from either a combination or monotherapy study using doxorubicin at doses of 0, 4.2, 7.2, 12 mg/kg, and/or mIL-18 (SEQ ID NO:2) at doses of 0, 1, 5, 25 µg/mouse. The plot uses the maximum value of surface probability that corresponds to the treatment combination that minimizes the risk of death. This surface gives the predicted probability of survival for at least 30 days, at each treatment combination.

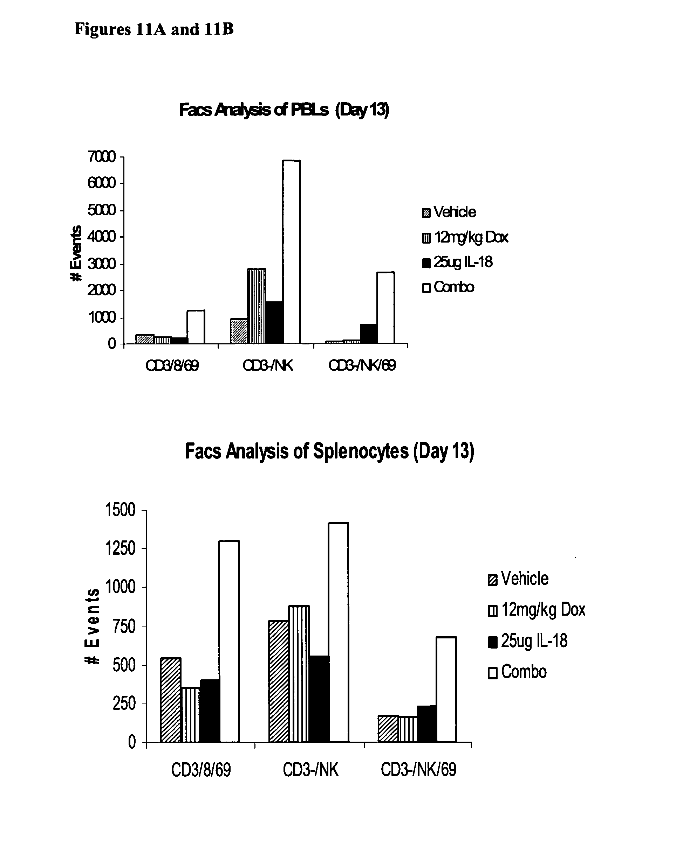

The effect on the immune cells in response to combination therapy of IL-18 and doxorubicin was addressed in a set of experiments that analyzed the viability, expansion, activation and functionality of the lymphocytes. The phenotypic profile of lymphocytes was measured in animals that were treated with either doxorubicin (12mg/kg), mIL-18 (SEQ ID NO:2) (25µg/dose), or by combination of both. The profile of activated CD8-postive T-cells, NKs and activated NKs was tested, and the data is shown in Figures 11A and 11B.

The combination of mIL-18 (SEQ ID NO:2) and doxorubicin increased/maintained the same number of activated CD8-positive T-cells (CTLs), NK and activated NK cells as doxorubicin alone. These cells may play a key role in cell-mediated cytotoxicity (specific tumor killing). The enhancement of activated CD8-positive T-cells and NK cells, in response to doxorubicin/IL-18 combo, was more enhanced in circulating PBLs, as compared to splenocytes.

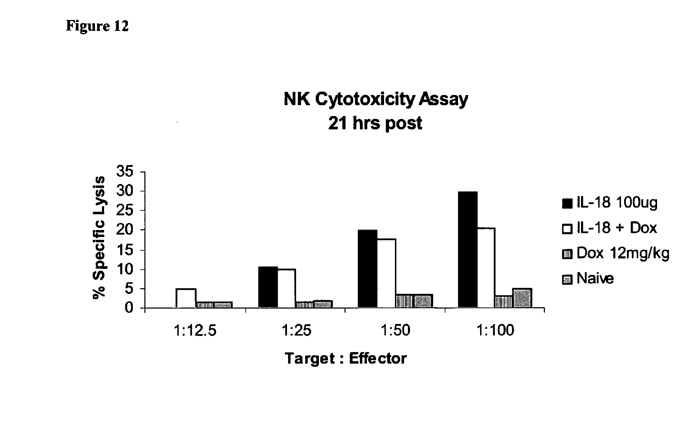

It was important to run an experiment to show that doxorubicin does not reduce IL-18 enhanced NK cell activity (non-specific tumor killing). Figure 12 demonstrates that NK cytotoxicity is impaired in animals just treated with doxorubicin, while animals that received mIL-18 (SEQ ID NO:2) alone, or combination with doxorubicin, both showed robust NK cytotoxicity.

### Example 3: Protocol for Phase I Clinical Trial of IL-18 combination with Rituximab

This Phase I is an open-label, dose-escalation study of human IL-18 in combination with standard Rituximab therapy investigating the safety and tolerability of 12 weekly ascending doses (1 to 100 µg/kg) of human IL-18 (SEQ ID NO:1) in subjects with CD20+ B cell NHL.

Dosing of Rituximab and human IL-18 (SEQ ID NO:1) is staggered. Therefore, subjects receive weekly IV infusions of Rituximab (375 mg/m²) on Day 1 of Weeks 1 to 4. Human IL-18 (SEQ ID NO:1) is administered as weekly IV infusions on Day 2 of Weeks 1 to 4 and on Day 2 (+/- 1 day) of Weeks 5 to 12. The starting dose of human IL-18 (SEQ ID NO:1) is 1 µg/kg, and dose escalation is planned to proceed to a nominal maximum dose of 100 µg/kg.

Dosing within each cohort is staggered with one subj ect receiving the first dose of Rituximab on Day 1 and human IL-18 (SEQ ID NO:1) on Day 2 and then monitored in-house for at least 24 hrs. If there are no safety or tolerability concerns, the next subjects within the cohort is dosed at least 24 hrs later and will also be monitored in-house for 24 hrs after their first human IL-18 (SEQ ID NO:1) dose. On subsequent weeks (Weeks 2 to 12), subjects is monitored for 6 hrs after the human IL-18 dose and then may be released from the clinic. All subjects is dosed at least 2 hrs apart. No more than two subjects per day may be dosed in any cohort.

Three subjects are treated at the first dose level (1 µg/kg/week). If there is no evidence of toxicity greater than Grade 2 with "suspected" or "probable" relationship to study drug after completion of dosing in the cohort (i.e., all three subjects have completed Weeks 1 to 6 of study), three subjects are treated in each subsequent cohort at the following dose levels: 3 µg/kg/week, 10 µg/kg/week, 20 µg/kg/week, 30 µg/kg/week, and 100 µg/kg/week.

For all infusions of Rituximab, the complete delivery of the dose, from the initiation of infusion to the end of infusion, must not be less than 4 hrs. Human IL-18 infusion takes place over a two-hour period.

The goal of this study is to determine the maximal biologically effective dose of human IL-18 that is safe when used in combination with standard Rituximab treatment in subjects with CD20+ B cell lymphoma. In order to evaluate the dose-response relationship for human IL-18 (SEQ ID NO:1), which was found to be bell-shaped in previous Phase I studies, a dose range of 1 to 100 µg/kg will be used to examine the lower (low dose) and upper end (mid-range or high dose) of the biologically active range in subjects with CD20+ B cell lymphoma.

The dose of Rituximab is the standard regimen recommended in the approved labelling for patients with CD20+ B cell NHL. Doses of human IL-18 (SEQ ID NO:1) are selected based on previous Phase I safety, pharmacokinetic, and pharmacodynamic data from studies involving patients with renal cell carcinoma and metastatic melanoma. The dose of Rituximab to be used in this study is the standard regimen recommended in the approved labelling for patients with CD20+ B cell NHL.

Doses of human IL-18 (SEQ ID NO:1) were selected based on previous Phase I safety, pharmacokinetic, and pharmacodynamic data from studies involving patients with renal cell carcinoma and metastatic melanoma. Robertson, et al., Proc. Am. Soc. Clin. Oncol. 22:178 (abstract 713) (2003); Robertson, et al., J. Clin. Oncol. 22:176s (abstract 2553) (2004); Robertson, et al., J. Clin. Oncol. 23:169s (abstract 2513) (2005); Koch, et al., J. Clin. Oncol. 23:174s (abstract 2535) (2005); Koch, et al., Eur. J. Cancer 4(12):86 (270) (2006). The highest dose tested, 2000 µg/kg administered weekly for up to 24 weeks, produced no significant toxicity such that a maximum tolerated dose was not identified; therefore, pharmacodynamic data were used to select the upper limit of the dose range for this study.

The highest dose tested, 2000 µg/kg administered weekly for up to 24 weeks, produced no significant toxicity such that a maximum tolerated dose was not identified; therefore, pharmacodynamic data are used to select the upper limit of the dose range for this study.

### Example 4: IL-18 and HERCEPTIN^{®} combination in mouse plasmacytoma model

Both the MOPC315.D3j005 and MOPC.D3j03 studies were analyzed to evaluate effect of murine IL-18 (SEQ ID NO:2) combination therapy with HERCEPTIN^{®} on the growth of murine plasmocytoma. For this experiment we had to transfect MoPC315 cells with ErbB2 (HER2). For transfection, we used 1.5ug ErbB2 expression vector (BioCat - 108912 - pcdna3.1(-) ErbB2 ) in a 6-well dish, as described using liposomal transfection with Lipofectamine™ and Optimem™ media from Gibco. Selective pressure neomycin (450ug/ml G418 Sigma G6816) was added to the cultures after 2 days. Initial positive populations were selected by fluorescent microscopic inspection of *in situ* cultures stained with Alexafluor488 labeled HERCEPTIN^{®} monoclonal antibody and cloned by limiting dilution (206434 p 70-72). D3 ErbB2 expression tested with Alexafluor488 labeled HERCEPTIN^{®} flow cytometry. MOPC.D3 cell line was selected and used for evaluation of HERCEPTIN^{®} and IL-18 anti-tumor efficacy.

The anti-tumor activity was measured and detailed analysis of the data revealed that the combinational therapy with IL-18 and HERCEPTIN^{®} surpasses the monotherapy with HERCEPTIN^{®} alone. Notably, this difference is statistically significant and robust; it was determined using non-parametric tests which are less sensitive, and less powerful in determining statistical difference.

A detailed analysis of the data revealed that the combinational therapy with IL-18 and HERCEPTIN^{®} surpasses the monotherapy with HERCEPTIN^{®} alone. Notably, this difference is statistically significant and robust; it was determined using non-parametric tests which are less sensitive, and less powerful in determining statistical difference.

### a. Study # MOPC315.D3j005

This study employed the combination of mIL-18 (SEQ ID NO:2) and HERCEPTIN^{®}, an anti-Her2/neu receptor antibody, with the goal to use this therapy in breast cancer in a clinical trial. Combination therapy was tested in the well established murine plasmocytoma cell line, MOPC315. The tumor line was obtained from ATCC and transduced with the Her2 receptor in-house. This tumor line is a BALB/c syngeneic cell line. The administration was as follows: murine IL-18 (SEQ ID NO:2) (100 µg/mouse q.d., s.c.), HERCEPTIN^{®} (200, 100 or 50 µg µg/mouse, twice a week, i.v.). The treatment in MOPC315.D3j005 study was initiated after the tumors started to grow, which was on day 14 after implantation.

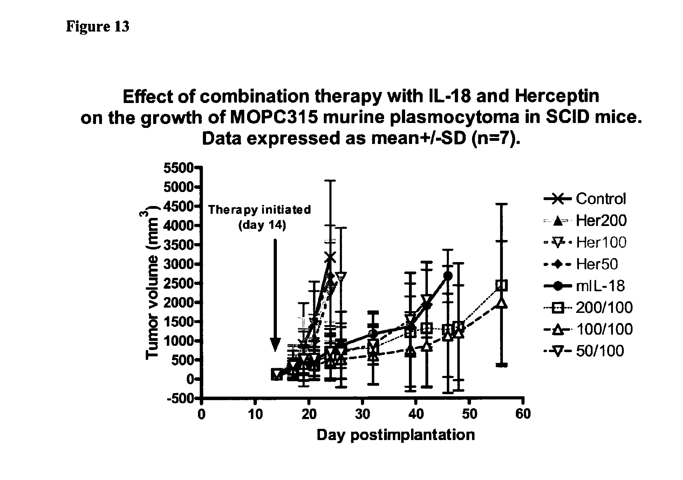

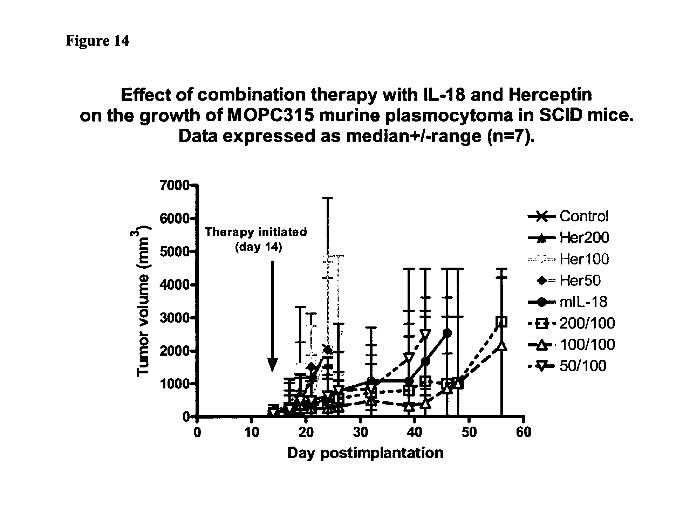

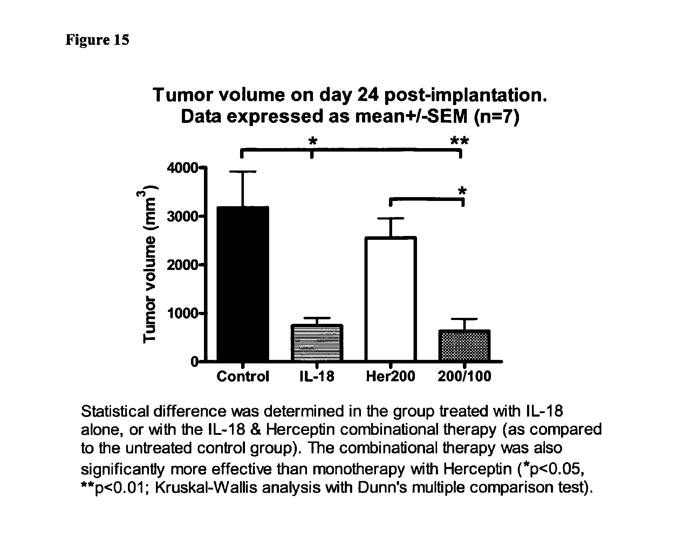

The results of this study are shown in Figures 13 and 14, expressing the data as mean+/-SD (Figure 13) and as median+/-range (Figure 14). We first checked to verify whether the data follow normal distribution (Gaussian approximation), and we compared standard deviation values to make sure that there is an equal variance (viz Figures 14 and 15). We found that there is a normal distribution of the raw data, however the standard deviation between the treatment groups is highly variable (>3x), and, therefore, we cannot use the parametric test (such as ANOVA) for analysis. We transformed the data using log10 and In to see if the transformed data pass the normality and equal variance tests (sample analysis displayed below ― for select groups on day 24). The transformed data did not pass the normality test. Therefore, we chose a non-parametric test (Kruskal-Wallis analysis) for the statistical evaluation. The detailed data and p values are displayed in Figures 13 and 14.

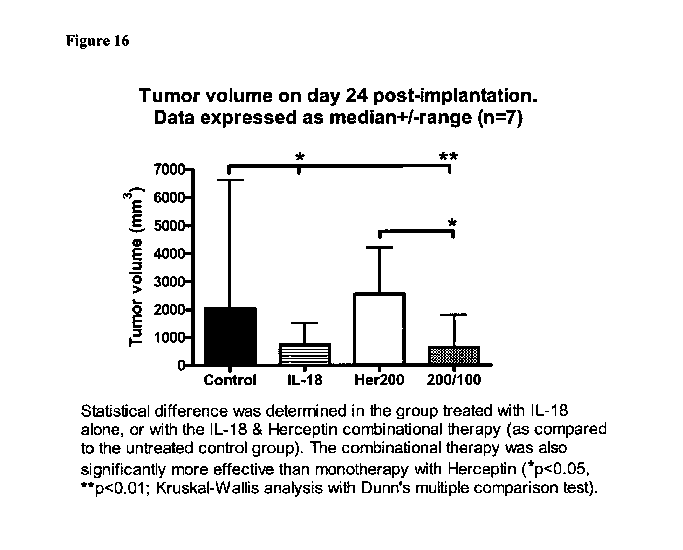

The statistical analysis revealed that combination therapy with mIL-18 (SEQ ID NO:2) and HERCEPTIN^{®} is better than monotherapy with HERCEPTIN^{®} alone. Figure 15 shows the statistical difference (Kruskal-Wallis analysis, p<0.05) between the group dosed with HERCEPTIN^{®} 200 µg/mouse alone, and the group treated with both HERCEPTIN^{®} 200 µg/mouse, and human IL-18 100 µg/mouse. Figure 16 shows that the combination treatment with HERCEPTIN^{®} and IL-18 showed the best window of anti-tumor activity, as compared to either HERCEPTIN^{®} and IL-18 alone.

### b. Study # MOPC.D3J03

This study was identical to the MOPC315.D3J005 study above (Example 4.a.) with the exception that the therapy started before the tumors became macroscopically apparent, on day 7 post-implantation. In addition, the maximal dose of HERCEPTIN was 100 µg/mouse, and the minimal dose was 25 µg/mouse in this study.

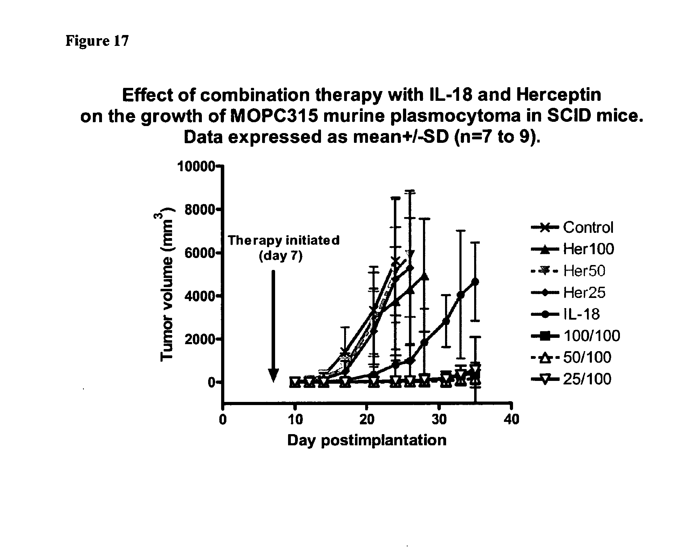

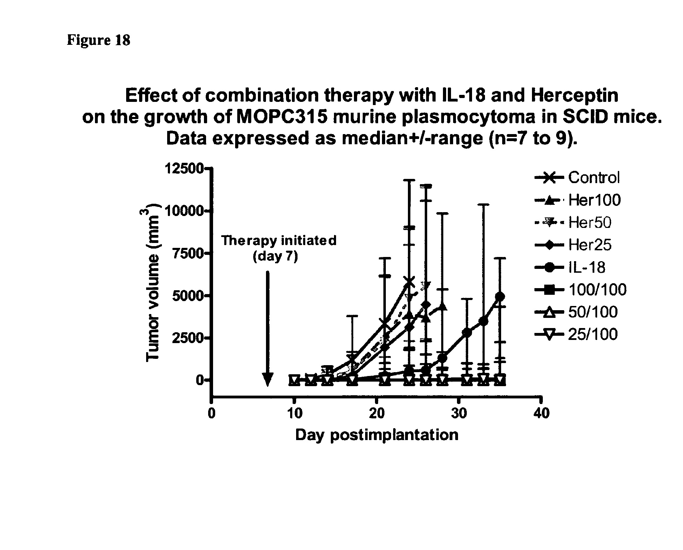

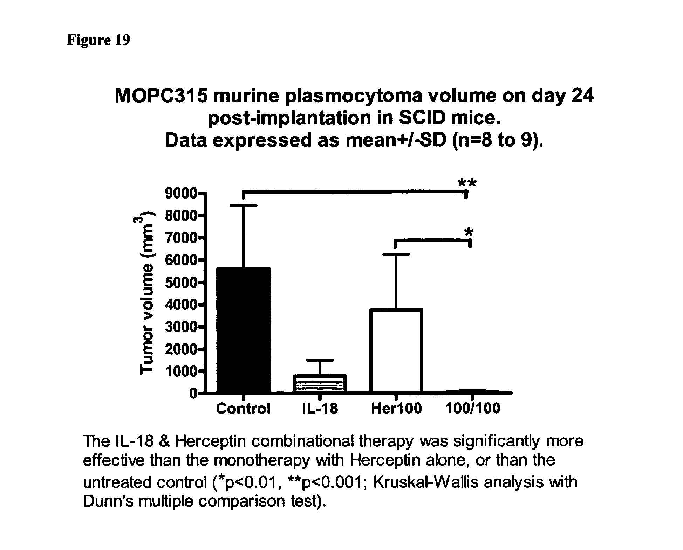

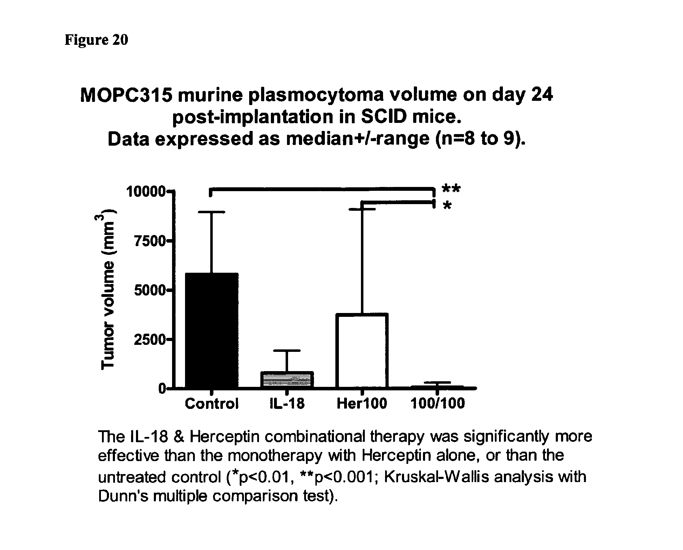

The data are expressed as mean+/-SD (Figure 17), and as median+/-range (Figure 18). We first checked if the data follow normal distribution (Gaussian approximation), and we compared standard deviation values to make sure that the equal variance test passes. We found that the raw data do not follow normal distribution; also the transformed data (log10 or In) did not follow Gaussian distribution, nor did they pass an equal variance test. Therefore we could not use a parametric test (such as ANOVA), and we chose a non-parametric test (Kruskal-Wallis analysis) for the statistical evaluation. The detailed data and p values are displayed below in Figures 19 and 20.

In conclusion, the statistical analysis of this study revealed that combination therapy with mIL-18 (SEQ ID NO:2) and HERCEPTIN^{®} is better than monotherapy with HERCEPTIN^{®} alone. The graphs in Figures 19 and 20 show the significantly better regression of the tumor in the combination therapy group (mIL-18 (SEQ ID NO:2) 100 µg/mouse and HERCEPTIN^{®} 100 µg/mouse), as compared with the monotherapy with HERCEPTIN^{®} alone (100 ®g/mouse). HERCEPTIN^{®} as monotherapy has minimal activity that is, however, augmented by IL-18 combination treatment. Since HER2 is transfected into cells, HERCEPTIN^{®} can only provide binding to HER2, but no induction of apoptosis (tumor cell death). Therefore, the anti-tumor activity is a result of combo therapy, where IL-18 is augmenting cells that play key role in ADCC and CDC activity (cells that are augmented by IL-18 treatment) and HERCEPTIN provides the specific binding to HER2 and serves as ADCC/CDC target.

### Example 5: Analysis of the IL-18 & 5-fluorouracil (5-FU) combination therapy in the syngeneic model of murine colon cancer, Colo26

This study aimed to evaluate the efficacy ofmIL-18 (SEQ ID NO:2) combination therapy with 5-fluorouracil (5-FU), as compared to monotherapy with 5-FU, or mIL-18 (SEQ ID NO:2) alone. Our study was performed in a well established syngeneic subcutaneous model of murine colon carcinoma, Colo 26, in BALB/c mice. The dosing with mIL-18 (SEQ ID NO:2) was performed daily with 10 µg/mouse s.c. on days 10 ― 30 after tumor inoculation. The dosing with 5-FU was performed i.p. twice a week in the ascending dose: 27, 45 and 74 µg/mouse.

A detailed analysis of the tumor volume data revealed that the combinational therapy with 10 µg of mIL-18 (SEQ ID NO:2) and 75 µg of 5-FU is the only treatment group with the significant effect on tumor growth, as compared to the control group. This means that the combination therapy (75 µg /10 µg) surpassed the monotherapy groups with 5-FU alone, or with mIL-18 (SEQ ID NO:2) alone, because monotherapy did not show a therapeutic effect better than a control. It is important to know that this difference is statistically significant and robust ― it was determined using non-parametric tests which are less sensitive, and less powerful in determining statistical difference. In addition, survival analysis demonstrated that the combination therapy (75 µg /10 µg) was significantly better than the monotherapy group (75 µg). The significance was extremely strong with p<0.0001.

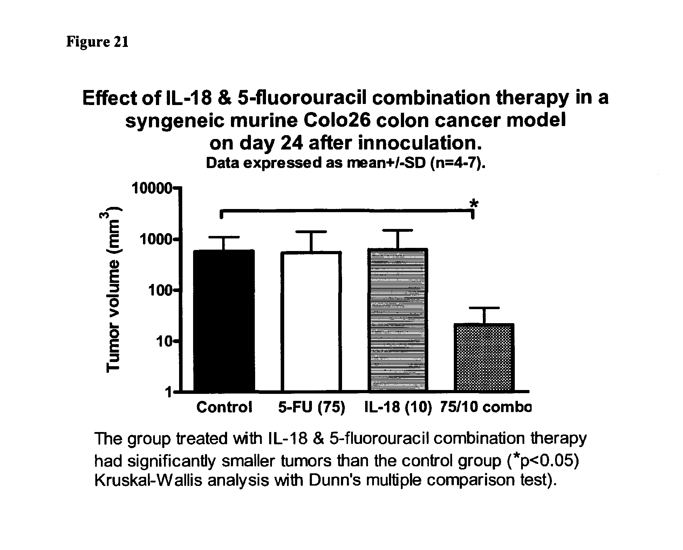

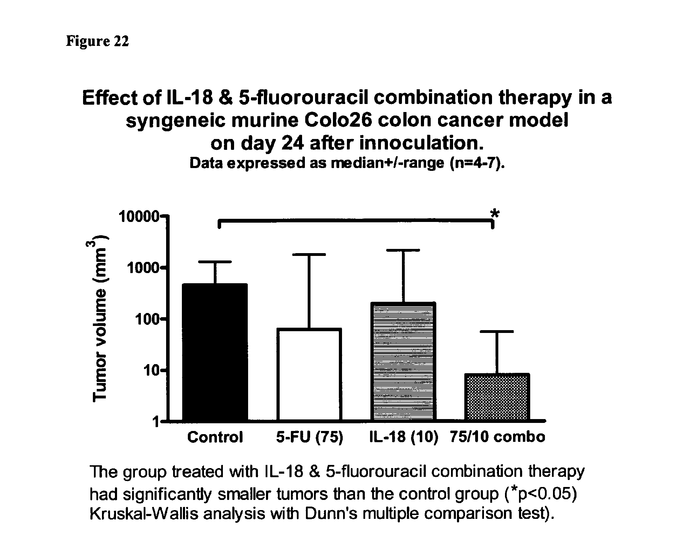

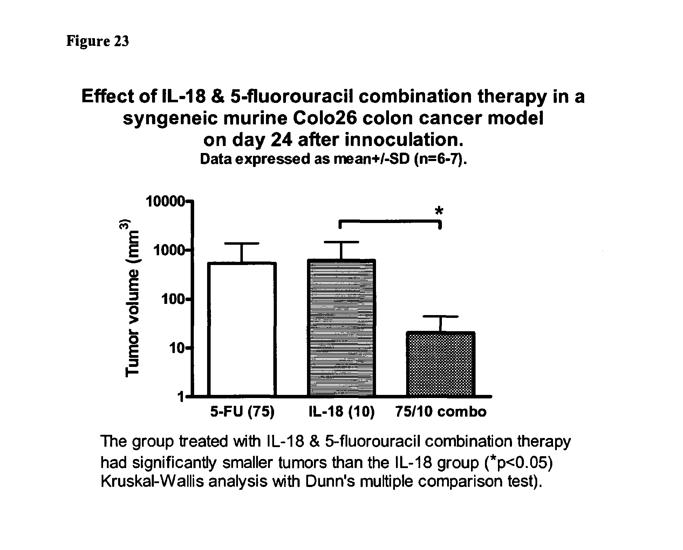

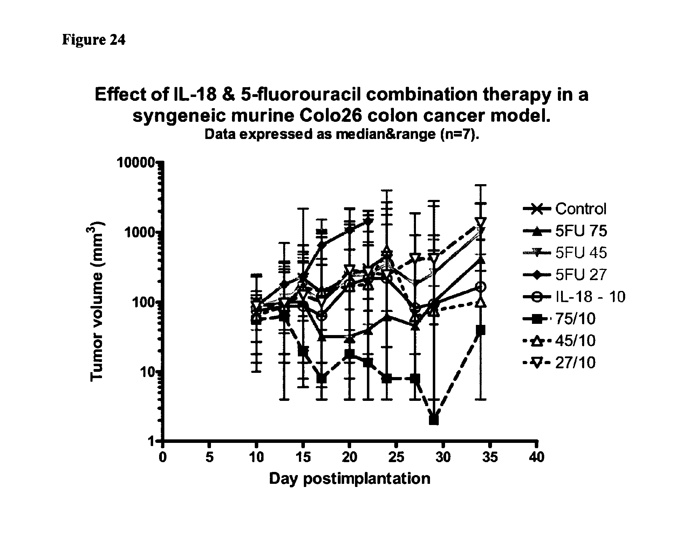

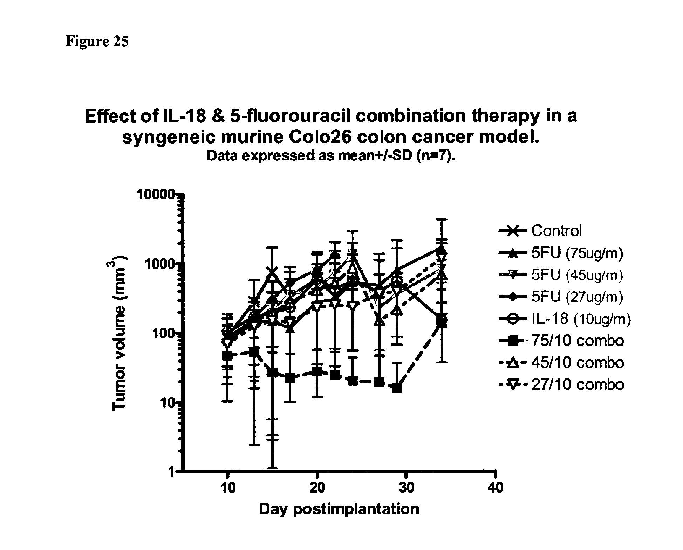

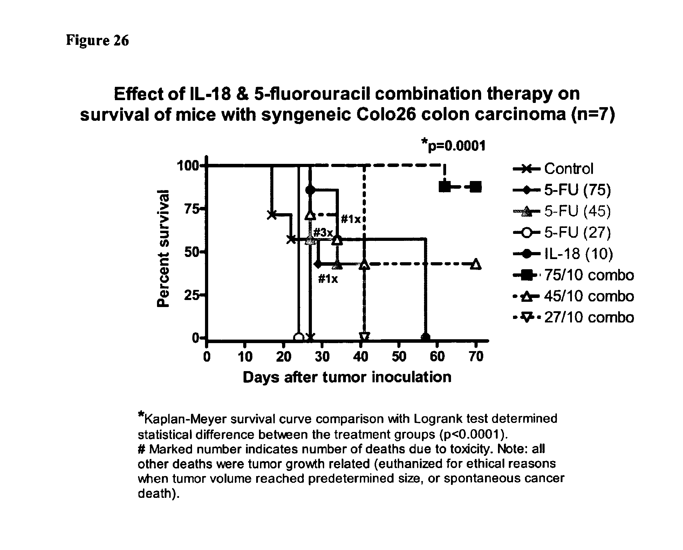

The data comparing tumor volumes in different treatment groups were evaluated at a selected representative time-point, and were expressed as mean+/-SD (Figure 21), and as median+/-range (Figure 22). The data were first checked for normal distribution (Gaussian approximation), and standard deviation values were compared to make sure that there is an equal variance. However, the distribution of some of the raw data did not follow Gaussian curve, also the standard deviation between the treatment groups was highly variable (>3x) and therefore the parametric test could not be used for analysis. The data were transformed using log 10, and they still did not pass the normality and equal variance tests (sample analysis displayed below ― for select groups). Therefore, a non-parametric test (Kruskal-Wallis analysis and Dunn's comparison test) was used for the statistical evaluation. The detailed data and p values are displayed in the graphs below in Figure 23. Figure 24 (median +/-SD) and Figure 25 (mean +/-SD) show the effect of IL-18 and 5-FU combination therapy in same Colo26 syngeneic colon tumor model. It is clear that animals were treated when the tumor volume reached between 80-100 cu mm size (advanced tumor model), either with IL-18 alone, 5-FU alone or in combination of both drugs. The better view of anti-tumor activity and synergy for combination treatment are presented in Figure 26.

Survival of mice bearing Colo26 in different treatment groups was plotted in a Kaplan-Meyer survival curve analysis, and evaluated by Logrank test, and is shown in Figure 26. There was a statistical difference in survival between the treatment groups with the best group being the combination therapy with 10 µg of mIL-18 (SEQ ID NO:2) and 75 µg of 5-FU.

### Example 6: Efficacy of combination therapy with IL-18 and pazopanib (GW786034) in mouse renal cell carcinoma model

This study, the RENJ02 study, tested the efficacy of combination therapy with mIL-18 (SEQ ID NO:2) and pazopanib, an inhibitor of VEGFR & PDGFR & c-kit tyrosine kinases in the advanced syngeneic model of mouse renal carcinoma. This animal model is a murine subcutaneous solid renal carcinoma model. Murine RENCA cell line syngeneic with BALB/c mice was implanted in BALB/c recipients. The dosing schedule employed is depicted below in the Table 1. IL-18 was dosed once a day on days 14 to 42 s.c. Pazopanib was dosed once a day on days 14 to 42 p.o.

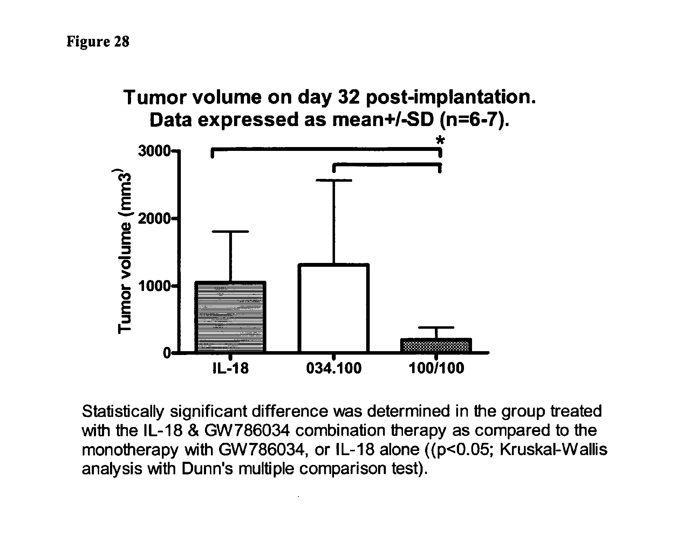

First, for statistical analysis, a good time-point for comparisons between the groups was determined. Then, the data were subjected to normality testing to determine a suitable statistical test for analysis. Day 32 was chosen as a representative time-point (some mice had to be euthanized for toxicity or tumor size by this time-point, therefore groups show 5-7 mice, although originally each group started with 7 mice). The data did not show a Gaussian (normal) distribution and therefore a non-parametric test was used. A statistical difference between monotherapy and combination therapy was determined, and is shown in Figure 28, even though a non-parametric test had to be used (has lower power to detect difference, than parametric). Statistical software used for evaluation included Prism GraphPad and SigmaStat.

**Table 1**

| *Group* | *# of mice* | *pazopanib(µg)* | *IL-18 (µg)* |
|---|---|---|---|
| 1 | 7 | **10** | **0** |
| 2 | 7 | **30** | **0** |
| 3 | 7 | **100** | **0** |
| 4 | 7 | **10** | **100** |
| 5 | 7 | **30** | **100** |
| 6 | 7 | **100** | **100** |
| 7 | 7 | **0** | **100** |
| 8 | 7 | **0** | **0** |

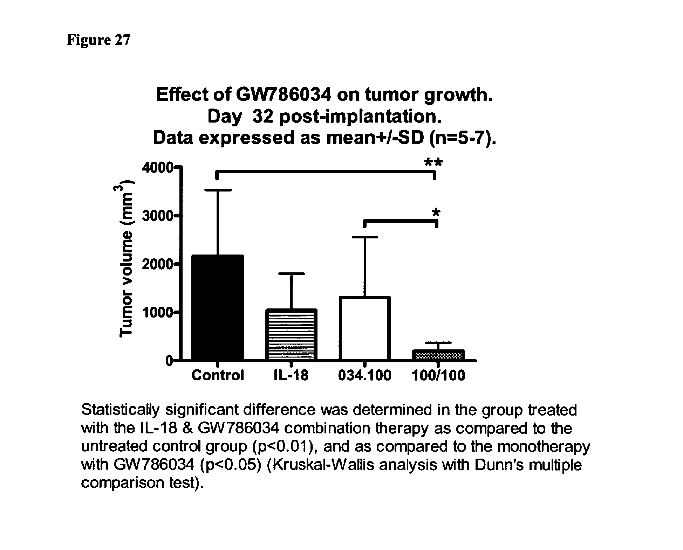

Figure 28 analyzes the same data as Figure 27. However, in Figure 28, the control group is not included. This additional graph was done to perform a "cleaner" analysis by comparing solely the monotherapy and combination therapy groups. These data show that combination treatment with pazopanib (GW786034) and IL-18 results in statistically significant anti-tumor activity.

### Example 7: Addressing the role of IL-18 as an inducer of memory that would result in long- term survival and prevention of tumor relapse.

We address this question by testing efficacy in EL-4 tumor model, where mice were treated by combination of murine IL-18 (SEQ ID NO:2) and doxorubicin. Those mice that were cured, when re-challenged with the tumor, were resistant to tumor take/growth, suggesting that they have memory mechanism that was induced by treatment of IL-18 and doxorubicin. The presence of T- memory cells in EL-4 tumor mice that survived, and their tumors were cured by IL-18 and doxorubicin treatment are presented in experiment below (Figures 29 and Figure 30).

This experimental design was as follows: *PfplRag2* mice (*H2b* haplotype with severe depletion in NK cell and CTL activity) received adoptive transfer of 2.5x10⁷ spleen and lymph node cells from the IL-2 (3000 U per mouse q.d., s.c.) treated survivors, or control C57BL/6 mice (both survivor and control mice received IL-2). Two weeks after adoptive transfer all mice were challenged with EL-4 tumor cells (EL-4 is a carcinogen induced mouse lymphoma of C57BL/6 (H2b) origin). All recipients were treated with IL-2 (3000 U per mouse q.d., s.c.) for three days after adoptive transfer (starting on the day of adoptive transfer). The recipient strain was selected purposely to have the same genetic background as the innoculated tumor. Weight and survival of the mice was recorded to establish a time-line of weight loss/gain and the abdominal cavity was palpated to determine presence of palpable tumor mass during the weeks after EL-4 innoculation.

The *PfplRag2* mice were purchased in the maximum quantities available ― 4 males and 4 females. We also had two older mice left from the previous study. In order to increase numbers of samples per group as much as possible, we decided to utilize all these mice: to avoid effects of sex and age on the results, the sexes and age were evenly distributed between the two groups: one received the lymphatic cells from EL-4 survivors, and the other received cells from the normal control B6 mice.

Results indicated that there was no significant difference in weight between the two groups of mice during the first week after EL-4 challenge, and the weight gain in all mice stagnated (Figure 29). This results may be due to the fact that all mice were receiving IL-2 s.c. to boost their immune response during the first three days. During the second and third week after EL-4 challenge, we observed a rapid weight gain and palpable tumor and/or ascites formation in the control group. All control mice died within two weeks, however all survivor-cell recipients survived although two (out of 5) had a palpable tumor in the abdomen. One mouse had to be euthanized, due to rapid growth of the tumor for ethical reasons.

**Table 2**

| **Adoptive transfer from (IL-2 s.c. 3000 IU/m 3 days)** | **Number of cells** | **Recipients treated with IL-2 s.c.3000 IU/m 3 days** | **Susceptibility to EL-4** |
|---|---|---|---|
| IL-18 & Dox treated C57BL/6 survivors | 2.5x10e7 | yes | Protected |
| Normal C57BL/6 mice | 2.5x10e7 | yes | Not protected |

Table 2 shows the summary of the findings with respect to protection against tumor challenge in mice that were IL-18/doxorubicin treated, versus normal control animals. Mice that received lymphatic cells from IL-18/doxorubicin treated animals were protected, while lymphatic cells from control animals showed tumor take/growth.

The EL-4 recipient mice that received survivor lymphatic cells survived significantly longer than control mice that received lymphatic cells from normal naive donors. The data imply that the adoptive transfer from survivor mice had a protective effect on the EL-4 tumor recipients. These data offer an indirect demonstration of memory T cells in the EL-4 tumor survivors (Figure 29 and Figure 30).

This is an important finding that could make combination of any chemotherapeutic agent or mAb with IL-18 a superior cancer treatment to any monotherapy. Induction of memory T-cells that could recognize tumor as "foreign" and prevent relapse would be highly beneficial, and IL-18 with its good safety profile, a drug for any potential combination therapy.

The invention comprises the following embodiments...
1. A method of treating cancer in a patient in need thereof, comprising the step of: separately administering to the patient a composition comprising: (i) a human IL-18 polypeptide (SEQ ID NO:1) in combination with a carrier and; and (ii) a monoclonal antibody against an antigen that is expressed on the surface of a cancer cell, wherein the antibody has antibody-dependent-cell-mediated cytoxicity (ADCC) effector function, and wherein the antibody is not an anti-CD20 antibody.
2. The method as stated in embodiment 1, wherein the administration of the composition comprising the human IL-18 polypeptide (SEQ ID NO:1) and the monoclonal antibody is simultaneous.
3. The method as stated in embodiment 1, wherein the administration of composition comprising the human IL-18 polypeptide (SEQ ID NO:1) and monoclonal antibody is sequential, and wherein the human IL-18 polypeptide (SEQ ID NO:1) is administered first.
4. The method as stated in embodiment 1, wherein the administration of the composition comprising the human IL-18 polypeptide (SEQ ID NO:1) and antibody is sequential, and the monoclonal antibody is administered first.
5. The method as stated in embodiment 1, wherein the antigen is chosen from the group of: CD22, CD19, HER2, HER3, EGFR, IGF-1R, AXL-1, FGFR, integrin receptors, CEA, CD44, and VEGFR.
7. The method as stated in embodiment 5, wherein the antigen is HER-2, and the monoclonal antibody is HERCEPTIN^{®}
8. The method as stated in embodiment 1, wherein the cancer is chosen from the group of: Hodgkin's lymphoma, B-cell non-Hodgkin's lymphoma, Burkitt lymphoma, T-cell Non-Hodgkin's lymphoma, AML, CLL, MM, other leukemias, ovarian cancer, breast cancer, lung cancer, sarcoma, bladder cancer, pancreatic cancer, thyroid cancer, hepatoma, gastric cancer, Wilms', neuroblastoma, glioblastoma and other brain tumors, colon cancer, rectal cancer, prostate cancer, melanoma, renal cell carcinoma and skin cancers.
9. A method of treating cancer in a patient in need thereof, comprising the step of: separately administering to the patient a composition comprising: (i) human IL-18 polypeptide (SEQ ID NO:1) in combination with a carrier; and (ii) a chemotherapeutic agent.
10. The method as stated in embodiment 9, wherein the administration of composition comprising the human IL-18 polypeptide (SEQ ID NO:1) and the chemotherapeutic agent is simultaneous.
11. The method as stated in embodiment 9, wherein the administration of the composition comprising the human IL-18 polypeptide (SEQ ID NO:1) and the chemotherapeutic agent is sequential, and wherein the human IL-18 polypeptide (SEQ ID NO:1) is administered first.
12. The method as stated in embodiment 9, wherein the administration of the composition comprising the human IL-18 polypeptide (SEQ ID NO:1) and the chemotherapeutic agent is sequential, and wherein the chemotherapeutic agent is administered first.
13. The method as stated in embodiment 9, wherein the chemotherapeutic agent is chosen from the group of: doxil, topotecan, DNA-altering drugs, carboplatin, antimetabolites, gemcitabine, drugs that prevent cell division, vincristine, anti-angiogenic agents, and pazopanib.
14. The method as stated in embodiment 9, wherein the cancer is chosen from the group of: Hodgkin's lymphoma, B-cell non-Hodgkin's lymphoma, Burkitt lymphoma, T-cell Non-Hodgkin's lymphoma, AML, CLL, MM, other leukemias, ovarian cancer, breast cancer, lung cancer, sarcoma, bladder cancer, pancreatic cancer, thyroid cancer, hepatoma, gastric cancer, Wilms', neuroblastoma, glioblastoma and other brain tumors, colon cancer, rectal cancer, prostate cancer, melanoma, renal cell carcinoma, and skin cancers.
15. A method of treating cancer in a patient in need thereof, said method comprising the step of administering to the patient a composition comprising:
   human IL-18 (SEQ ID NO:1) in combination with a chemotherapeutic agent,
   whereby the treatment results in long-term survival and/or prevention of cancer reoccurrence and induction of immunological memory in the patient.
16. The method as stated in embodiment 15, wherein the chemotherapeutic agent is chosen from the group of: doxil, topotecan, DNA-altering drugs, carboplatin, antimetabolites, gemcitabine, drugs that prevent cell division, vincristine, anti-angiogenic agents, and pazopanib.
17. The method as stated in embodiment 15, wherein the cancer is chosen from the group of: Hodgkin's lymphoma, B-cell non-Hodgkin's lymphoma, Burkitt lymphoma, T-cell Non-Hodgkin's lymphoma, AML, CLL, MM, other leukemias, ovarian cancer, breast cancer, lung cancer, sarcoma, bladder cancer, pancreatic cancer, thyroid cancer, hepatoma, gastric cancer, Wilms', neuroblastoma, glioblastoma and other brain tumors, colon cancer, rectal cancer, prostate cancer, melanoma, renal cell carcinoma, and skin cancers.
18. A method of treating cancer in a patient in need thereof, said method comprising the step of administering to the patient a composition comprising:
   human IL-18 (SEQ ID NO:1) in combination with a monoclonal antibody against an antigen that is expressed on the surface of a cancer cell, wherein the antibody has antibody-dependent-cell-mediated cytoxicity (ADCC) effector function, and wherein
   the antibody is not an anti-CD20 antibody. whereby the treatment results in long-term survival and/or prevention of cancer reoccurrence and induction of immunological memory in the patient.
19. The method as stated in embodiment 18, wherein the antigen is chosen from the group of: CD22, CD19, HER2, HER3, EGFR, IGF-1R, AXL- 1, FGFR, integrin receptors, CEA, CD44, and VEGFR.
20. The method as stated in embodiment 18, wherein the cancer is chosen from the group of: Hodgkin's lymphoma, B-cell non-Hodgkin's lymphoma, Burkitt lymphoma, T-cell Non-Hodgkin's lymphoma, AML, CLL, MM, other leukemias, ovarian cancer, breast cancer, lung cancer, sarcoma, bladder cancer, pancreatic cancer, thyroid cancer, hepatoma, gastric cancer, Wilms', neuroblastoma, glioblastoma and other brain tumors, colon cancer, rectal cancer, prostate cancer, melanoma, renal cell carcinoma, and skin cancers.

## Claims

1. A composition comprising human IL-18 polypeptide (SEQ ID NO:1) and pazopanib for use in treating cancer.

2. A composition comprising human IL-18 polypeptide (SEQ ID NO:1) in combination with a carrier for use in treating cancer in a patient in need thereof, wherein said composition is to be used in combination with pazopanib.

3. Pazopanib for use in treating cancer in a patient in need thereof, wherein pazopanib is to be used in combination with a composition comprising a human IL-18 polypeptide (SEQ ID NO:1) in combination with a carrier.

4. Use of a composition comprising human IL-18 polypeptide (SEQ ID NO:1) in the manufacture of a medicament for use combination with pazopanib for treating cancer.

5. Use of a pazopanib in the manufacture of a medicament for use combination with a composition comprising human IL-18 polypeptide (SEQ ID NO:1) for treating cancer.

6. The composition as claimed in claim 2, pazopanib as claimed in claim 3, or the use as claimed in claim 4 or 5, wherein the use of the composition comprising human IL-18 polypeptide (SEQ ID NO:1) and pazopanib is to be simultaneous.

7. The composition as claimed in claim 2, pazopanib as claimed in claim 3, or the use as claimed in claim 4 or 5, wherein the use of the composition comprising human IL-18 polypeptide (SEQ ID NO:1) and pazopanib is to be sequential, and wherein the human IL-18 polypeptide (SEQ ID NO:1) is to be used first.

8. The composition as claimed in claim 2, pazopanib as claimed in claim 3, or the use as claimed in claim 4 or 5, wherein the use of the composition comprising human IL-18 polypeptide (SEQ ID NO:1) and pazopanib is to be sequential, and wherein pazopanib is to be used first.

9. A composition as claimed in claim 1, whereby the treatment results in long-term survival and/or prevention of cancer reoccurrence and induction of immunological memory in the patient.

10. A composition as claimed in claim 1, 2 or 6-9, or pazopanib as claimed in claim 3 or 6-8, or the use as claimed in claim 4 or 5-9, wherein the cancer is chosen from the group of: Hodgkin's lymphoma, B-cell non-Hodgkin's lymphoma, Burkitt lymphoma, T-cell Non-Hodgkin's lymphoma, AML, CLL, MM, other leukemias, ovarian cancer, breast cancer, lung cancer, sarcoma, bladder cancer, pancreatic cancer, thyroid cancer, hepatoma, gastric cancer, Wilms', neuroblastoma, glioblastoma and other brain tumors, colon cancer, rectal cancer, prostate cancer, melanoma, renal cell carcinoma and skin cancers.


SEQUENCE LISTING

<110> HASKOVA, Zdenka
      JONAK, Zdenka L.
      TRULLI, Stephen H.
      WHITACRE, Margaret N.

<120> METHODS OF TREATING CANCER BY
  ADMINISTERING HUMAN IL-18 COMBINATIONS

<130> PU62371D1 EP

<140> 12/052,278
<141> 2008-03-20

<150> 60/952,002
<151> 2007-07-26

<150> 60/896,855
<151> 2007-03-23

<160> 2

<170> FastSEQ for Windows Version 4.0

<210> 1
<211> 157
<212> PRT
<213> Homo Sapien

<400> 1
Tyr Phe Gly Lys Leu Glu Ser Lys Leu Ser Val Ile Arg Asn Leu Asn
 1               5                  10                  15
Asp Gln Val Leu Phe Ile Asp Gln Gly Asn Arg Pro Leu Phe Glu Asp
            20                  25                  30
Met Thr Asp Ser Asp Cys Arg Asp Asn Ala Pro Arg Thr Ile Phe Ile
        35                  40                  45
Ile Ser Met Tyr Lys Asp Ser Gln Pro Arg Gly Met Ala Val Thr Ile
    50                  55                  60
Ser Val Lys Cys Glu Lys Ile Ser Thr Leu Ser Cys Glu Asn Lys Ile
65                  70                  75                  80
Ile Ser Phe Lys Glu Met Asn Pro Pro Asp Asn Ile Lys Asp Thr Lys
                85                  90                  95
Ser Asp Ile Ile Phe Phe Gln Arg Ser Val Pro Gly His Asp Asn Lys
            100                 105                 110
Met Gln Phe Glu Ser Ser Ser Tyr Glu Gly Tyr Phe Leu Ala Cys Glu
        115                 120                 125
Lys Glu Arg Asp Leu Phe Lys Leu Ile Leu Lys Lys Glu Asp Glu Leu
    130                 135                 140
Gly Asp Arg Ser Ile Met Phe Thr Val Gln Asn Glu Asp
145                 150                 155

<210> 2
<211> 157
<212> PRT
<213> Murine

<400> 2
Asn Phe Gly Arg Leu His Cys Thr Thr Ala Val Ile Arg Asn Ile Asn
 1               5                  10                  15
Asp Gln Val Leu Phe Val Asp Lys Arg Gln Pro Val Phe Glu Asp Met
            20                  25                  30
Thr Asp Ile Asp Gln Ser Ala Ser Glu Pro Gln Thr Arg Leu Ile Ile
        35                  40                  45
Tyr Met Tyr Lys Asp Ser Glu Val Arg Gly Leu Ala Val Thr Leu Ser
    50                  55                  60
Val Lys Asp Ser Lys Met Ser Thr Leu Ser Cys Lys Asn Lys Ile Ile
65                  70                  75                  80
Ser Phe Glu Glu Met Asp Pro Pro Glu Asn Ile Asp Asp Ile Gln Ser
                85                  90                  95
Asp Leu Ile Phe Phe Gln Lys Arg Val Pro Gly His Asn Lys Met Glu
            100                 105                 110

Phe Glu Ser Ser Leu Tyr Glu Gly His Phe Leu Ala Cys Gln Lys Glu
        115                 120                 125
Asp Asp Ala Phe Lys Leu Ile Leu Lys Lys Lys Asp Glu Asn Gly Asp
    130                 135                 140
Lys Ser Val Met Phe Thr Leu Thr Asn Leu His Gln Ser
145                 150                 155